# EUROPEAN PATENT SPECIFICATION

(11) **EP 0 606 463 B1**
(45) Date of publication and mention of the grant of the patent: **04.10.2001**
(21) Application number: 93918408.1
(22) Date of filing: 27.07.1993
(51) Int. Cl.: A61K 38/08, C07K 7/06, C07K 14/655

(54) **NEUROMEDIN B RECEPTOR ANTAGONISTS**
ANTAGONISTEN DES NEUROMEDIN B REZEPTORS
ANTAGONISTES DES RECEPTEURS DE LA NEUROMEDINE B

(30) Priority: 27.07.1992 US 919537; 17.06.1993 US 78419
(43) Date of publication of application: 20.07.1994
(73) Proprietor: The Administrators of The Tulane Educational Fund, New Orleans Louisiana 70112 (US); BIOMEASURE, INC., Hopkinton MA 01748 (US)
(72) Inventor: COY, David, H., New Orleans, LA 70115 (US); TAYLOR, John, E., Upton, MA 01568 (US)
(74) Representative: Deans, Michael John Percy
(86) International application number: US9307036
(87) International publication number: WO9402163

(56) References cited:
- EP-A- 0 215 171
- EP-A- 0 298 732
- EP-A- 0 395 417
- WO-A-89/04666
- WO-A-91/04040
- WO-A-91/09056
- HODGES E.A.: "Peptides, Chemistry, Structure and Biology; Proceedings 13th American Peptide Symposium, June 20-25, 1993, Edmonton, Alberta Canada" 1994 , ESCOM , LEIDEN XP002033293 Coy e.a.:"First neuromedin B receptor antagonists are based on somatostatin octapeptide analogues" * page 496 - page 498 *
- GASTROENTEROLOGY, vol. 104, no. 4(suppl), April 1993, page A845 XP000676395 M.ORBUCH E.A.: "Discovery of a novel class of NMB receptor antagonists: substituted somatostatin analogues"
- BIOCHEM.J., vol. 286, no. 2, 1 September 1992, pages 641-648, XP000676389 L-H. WANG E.A.: "Activation of neuromedin B preferring bombesin receptors on rat glioblastoma C-6 cells increase cellular Ca2+ and phosphoinositides"
- TRENDS PHARMACOL.SCI. , vol. 12, no. 1, January 1991, pages 13-19, XP000676386 R.T.JENSEN E.A.: "Progress in the development of potent bombesin receptor antagonists"

## Description

This invention relates to use of octapeptides in the treatment of diseases associated with Neuromedin B receptor.

The mammalian bombesin (Bn)-related peptides, gastrin-releasing peptide (GRP) and neuromedin B (NMB) have a wide range of biological and pharmacological effects. These include stimulation of the release of numerous gastrointestinal hormones and peptides, stimulation of exocrine gland secretion chemotaxis, contraction of smooth muscle, effects in the central nervous system such as thermoregulation, behavioral effects, maintenance of circadian rhythm, inhibition of TSH release and satiety. Bn-related peptides also function as a growth factor in numerous normal cells (e.g., bronchial cells, endometrial stomal cells and 3T3 cells) as well as neoplastic cells such as human small cell lung cancer cells, rat hepatocellular tumor cells, prostatic cells and breast adenocarcinoma cells.

Recent structure-function and cloning studies demonstrate that at least two classes of receptors mediate the actions of Bn-related peptides. One class, the GRP-preferring subtype (GRP receptor or GRP-R), has a high affinity for GRP and low affinity for NMB, whereas the other class, the NMB-preferring subtype (NMB receptor or NMB-R), has a high affinity for NMB and lower affinity for GRP. Both classes of receptors are widely present both in the central nervous system and in the gastrointestinal tract. Until recently, the physiological importance of Bn-related peptides in mediating various processes or which receptor subtype mediated the various reported biological effects of Bn-related peptides was unclear.

Five different classes of Bn-receptor antagonists have been described. Jensen, R. T. et al. *Trends* *Pharmacol. Sci.* 12:13 (1991). Members of a number of these classes have high potency, long duration of action and selectivity for the GRP receptor and thus are useful even *in vivo* for defining the role of GRP or GRP receptors in mediating various physiological events. However, at present, no antagonists for the NMB receptor which are sufficiently selective or potent have been described. Furthermore, when applied to NMB, none of the methodologies was used successfully to make potent selective GRP receptor antagonists such as synthesizing NMB pseudopeptides or desMet⁹ NMB or desMet⁹ NMB esters yields NMB receptor antagonists. Because of the absence of selective antagonists for NMB-R, it has been difficult to evaluate the physiological significance of NMB.

Recently, it was reported that a native somatostatin (SS), somatostatin-14 (SS-14), inhibited the cross-linking of ¹²⁵I-GRP to a 120 kD protein in triton extracts of 3T3 cells and human small cell lung cancer cells which are known to possess bombesin receptors. Recent studies have also demonstrated SS-14 could also weakly inhibit binding to opiate receptors, and subsequent structure-function led to the identification of various D-amino acid-substituted and constrained amino acid-substituted cyclo somatostatin analogs that functioned as potent mu opioid receptor antagonists.

In an Abstract published on page A845 of Gastroenterology, April 1993 between the two priority dates claimed for this application, the inventors for the present application together with co-workers reveal the somatostatin analogue D-Nal-Cys-Tyr-D-Trp-Lys-Val-Cys-Nal-NH₂ (Ss-octa) to interact with neuromedin B receptor.

In Molecular Pharmacology, Vol. 43, (1993), pp.838-844, which was also published between the two priority dates claimed for this application, the inventors for the present application and co-workers disclose certain somatostatin peptide analogues, including several octapeptide compounds. The affinities of these compounds to bind to three somatostatin receptor subtypes was investigated as well as their abilities to inhibit the release of growth hormone from anterior pituitary cells in vitro.

The following non-standard abbreviations are employed hereinbelow:
- Nal =: 3-(2-naphthyl)-alanine or 3-(1-naphthyl)-alanine
- Bpa =: 3-(4-biphenyl)-alanine
- X-Phe =: phenylalanine with a p-, o- or m-substituent X at its benzene ring, e.g., 3-(4-chlorophenyl)-alanine
- F₅Phe =: 3-(pentafluorophenyl)-alanine
- Nle =: norleucine
- Me-Trp =: Trp with its indolyl nitrogen substituted with methyl 
      R3
Lys(N<    ) = Lys with its ε amino group substituted with
      R4
        R3  and R4.  Thus, Lys(iPr) and Lys(diEt)
      stand for Lys residues with their ε amino groups
      monosubstituted by an isopropyl group and
      disubstituted by two ethyl groups,
      respectively.
      R3
Orn(N<    ) = Orn with its δ amino group substituted with
      R4
      R3 and R4
wherein
AA¹ is the D-isomer of an aromatic α-amino acid;
AA² is the L-isomer of Cys;
AA³ is Tyr;
AA⁴ is Trp;
AA⁵ is Lys;
AA⁶ is Leu, Ile, Nle, Val, Nal, Trp, Me-Trp, Bpa, F₅Phe, Phe, or 4-X-Phe in which X is halogen, NO₂, CH₃, or OH;
AA⁷ is the L-isomer of Cys;
AA⁸ is the L-isomer of an aromatic α-amino acid, Thr or Ser;
R₁ and R₂, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, C₁₂₋₂₀ naphthylalkynyl, COE, or CODE in which E is C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl, provided that when one of R₁ or R₂ is COE or COOE, the other must be H;
R₃ and R₄, independently, ₐᵣₑ H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; and
Y is OR₅ or HR₆R₇ in which each of R₅, R₆ and R₇, independently, is H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; provided that if AA⁸ is Thr or Ser, AA⁶ cannot be Val; for the manufacture of a medicament for use in the treatment of a disease which involves Neuromedin B receptor and does not involve somatostatin receptor.

In a second and alternative aspect of this invention, there is provided use of an octapeptide of the formula: 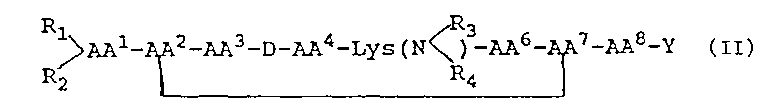 wherein
AA¹ is the D-isomer of an aromatic α-amino acid;
AA² is the L-isomer of Cys;
AA³ is Tyr;
AA⁴ is Trp;
AA⁶ is Thr or Ser;
AA⁷ is the L-isomer of Cys;
AA⁸ is the L-isomer of an aromatic α-amino acid;
R₁ and R₂, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, C₁₂₋₂₀ naphthylalkynyl, COE, or COOE in which E is C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl, provided that when one of R₁ or R₂ is COE or COOE, the other must be H;
R₃ and R₄, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; and
Y is OR_{S} or NR₆R₇ in which each of R₅, R₆ and R₇, independently, is H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ napthylalkyl, C₁₂₋₂₀ napthylalkynyl or C₁₂₋₂₀ napthylalkenyl; for the manufacture of a medicament for use in the treatment of a disease which involves Neuromedin B receptor and does not involve somatostatin receptor.

The invention provides in a third alternative aspect thereof, use of a octapeptide of the formula: 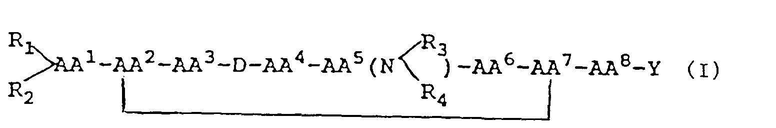 wherein
AA¹ is the D- or L-isomer of an aromatic α-amino acid;
AA² is the D- or L-isomer of Cys;
AA³ is F₅ Phe, Phe, or X-Phe in which X is halogen, NO₂, CH₃, or OH;
AA⁴ is Trp, or an aromatic α-amino acid;
AA⁵ is Lys or Orn;
AA⁶ is Leu, Ile, Nle, Val, Nal, Trp, Me-Trp, Bpa, F₅Phe, Phe, or X-Phe in which X is halogen, NO₂, CH₃, or OH;
AA⁷ is the D- or L-isomer of Cys;
AA⁸ is the D- or L-isomer of an aromatic α-amino acid, Thr or Ser;
R₁ and R₂, independently,are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, C₁₂₋₂₀ naphthylalkynyl, COE, or CODE in which E is C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl, provided that when one of R₁ or R₂ is COE or CODE, the other must be H;
R₃ and R₄, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; and
Y is OR₅ or NR₆R₇ in which each of R₅, R₆ and R₇, independently, is H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; provided that AA¹ and AA² cannot both be D-isomers; and further provided that if AA⁸ is Thr or Ser, AA⁶ cannot be Val; and excluding D-Nal-Cys-Tyr-D-Trp-Lys-Val-Cys-Nal-NH2(SS-octa) for the manufacture of a medicament for use in the treatment of a disease which involves Neuromedin B receptor and does not involve somatostatin receptor.

We provide, in a fourth alternative aspect of the present invention, use of an octapeptide of the formula: 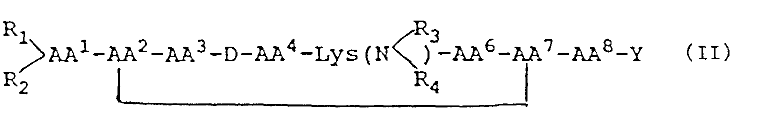 wherein
AA¹ is the D- or L-isomer of an aromatic α-amino acid;
AA² is the D- or L-isomer of Cys;
AA³ is F₅Phe, Phe, or X-Phe in which X is halogen, NO₂, CH₃, or OH;
AA⁴ is Trp, or an aromatic α-amino-acid;
AA⁶ is Thr or Ser;
AA⁷ is the D- or L-isomer of Cys;
AA⁸ is the D- or L-isomer of an aromatic α-amino acid;
R₁ and R₂, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, C₁₂₋₂₀ naphthylalkynyl, COE, or CODE in which E is C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl, provided that when one of R₁ or R₂ is COE or CODE, the other must be H;
R₃ and R₄, independently,ₐᵣₑ H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, naphthylalkyl, C₁₂₋₂₀ naphthylalkenyl, or C₁₂₋₂₀ naphthylalkynyl; and
Y is OR₅ or NR₆R₇ in which each of R₅, R₆ and R₇, independently, is H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ napthylalkyl, C₁₂₋₂₀. napthylalkenyl or C₁₂₋₂₀ napthylalkynyl; provided that AA¹ and AA² cannot both be D-isomers; for the manufacture of a medicament for use in the treatment of a disease which involves Neuromedin B receptor and does not involve somatostatin receptor.

Octapeptides which can be used to practice this invention include, but are not limited to:

H2-D-Nal-Cys-Tyr-D-Trp-Lys-Val-Cys-Nal-NH2 (Analog #1);
H2-D-Nal-Cys-Tyr-D-Trp-Lys-Nal-Cys-Thr-NH2 (Analog #2);
H2-D-Nal-Cys-Tyr-D-Trp-Lys-Nal-Cys-Nal-NH2 (Analog #3);
H2-D-Phe-Cys-Tyr-D-Trp-Lys-Val-Cys-Nal-NH2 (Analog #4);
H2-D-Nal-Cys-Tyr-D-Trp-Lys-Val-Cys-D-Nal-NH2 (Analog #8);
H2-Nal-Cys-Tyr-D-Trp-Lys-Val-Cys-D-Nal-NH2 (Analog #9);
H2-D-Nal-D-Cys-Tyr-D-Trp-Lys-Val-Cys-Nal-NH2 (Analog #10);
H2-D-Nal-Cys-Tyr-D-Trp-Lys-Val-D-Cys-Nal-NH2 (Analog #11);
H2-D-Trp-Cys-Tyr-D-Trp-Lys-Val-Cys-Nal-NH2 (Analog #12);
H2-D-Nal-Cys-Tyr-D-Trp-Lys-Phe-Cys-Nal-NH2 (Analog #13);
H2-D-Nal-Cys-Tyr-D-Nal-Lys-Val-Cys-Nal-NH2 (Analog #17);
H2-D-Phe-Cys-Tyr-D-Trp-Lys-Nal-Cys-Thr-NH2; and

H2-D-Nal-Cys-Tyr-D-Trp-Orn-Val-Cys-Nal-NH2.

Octapeptides which can be used to practice this invention also include, but are not limited to:

H2-D-Phe-Cys-Tyr-D-Trp-Lys-Thr-Cys-Nal-NH2 (Analog #7);
H2-D-Phe-Cys-Tyr-D-Trp-Lys(iPr)-Thr-Cys-Nal-NH2 (Analog
#15); H2-D-Phe-Cys-Tyr-D-Trp-Lys(diEt)-Thr-Cys-Nal-NH2
(Analog #16); H2-D-Phe-Cys-Tyr-D-Trp-Lys-Ser-Cys-Thr-NH2;
H2-D-Nal-Cys-Tyr-D-Trp-Lys-Thr-Cys-Nal-NH2; H2-D-Nal-D-
Cys-Tyr-D-Trp-Lys-Thr-Cys-Nal-NH2; and H2-D-Nal-Cys-Phe-D-
Trp-Lys-Thr-Cys-Nal-NH2.

In the formulae, the N-terminus is at the left and the C-terminus at the right in accordance with the conventional representation of a polypeptide chain. The symbol AA¹, AA², or the like in a peptide sequence stands for an amino acid residue, i.e., =N-CH(R)-CO- when it is at the N-terminus or -NH-CH(R)-CO- when it is not at the N-terminus, where R denotes the side chain of that amino acid residue. Thus, R is -CH(CH₃)₂ for Val. Also, when the amino acid residue is optically active, it is the L-form configuration that is intended unless D-form is expressly designated.

Note that the two Cys residues (i.e., AA² and AA⁷) in formula (I) are linked together via a disulfide bond.

COE stands for 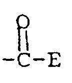 and CODE for

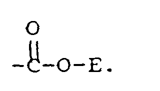

What is meant by "aromatic α-amino acid" is an amino acid residue of the formula 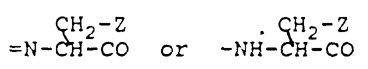 where Z is a moiety containing an aromatic ring. Examples of Z include, but are not limited to, a benzene ring and the following structures with or without a substituent X on the aromatic ring (where X is halogen, NO₂, CH₃, or OH):

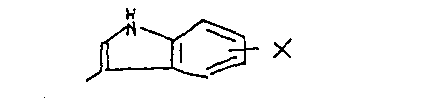

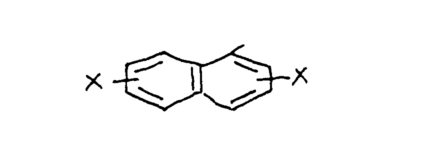

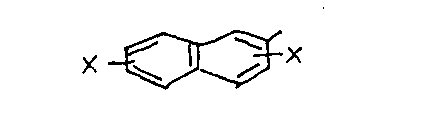

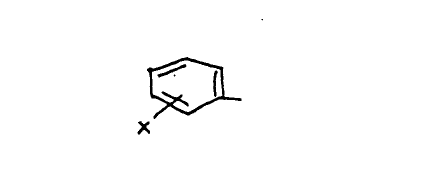

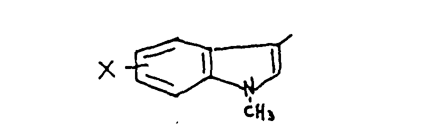

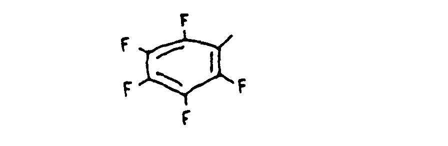

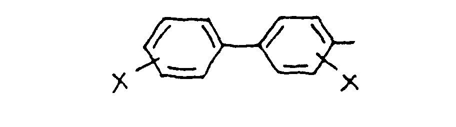

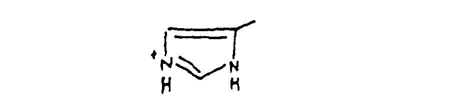

Other examples of an aromatic α-amino acid useful in practice of the invention are substituted His, such as MeHis, His (τ-Me), or His (π-Me).

The invention contemplates administration of a pharmaceutically acceptable salt of an octapeptide covered by the formulae into a patient whose disorder arises from biochemical activity induced by NMB. In other words, the octapeptides can be provided in the form of pharmaceutically acceptable salts, e.g., acid addition salts, or metal complexes, e.g., with zinc, iron or the like. Illustrative examples of acid addition salts are those with organic acids such as acetic, lactic, pamoic, maleic, citric, malic, ascorbic, succinic, benzoic, palmitic, suberic, salicylic, tartric, methanesulfonic or toluenesulfonic acid, those with polymeric acids such as tannic acid or carboxymethyl cellulose, and those with inorganic acids such as hydrochloric acid, hydrobromic acid, sulfuric acid or phosphoric acid.

The term "selectively" as recited in "selectively inhibiting biochemical activity of cells induced by neuromedin B" refers to preferred inhibition of NMB-stimulated production of inositol phosphates over GRP-stimulated amylase release. Such preference of the analogs used to practice the present invention is clearly shown in Table 2 below.

Other features and advantages of the present invention will be apparent from the following drawings and description of preferred embodiments,

The drawings are first described.

Figure 1 is a graph showing the ability of various somatostatin octapeptide analogs to inhibit binding of ¹²⁵I[D-Tyr⁰]NMB to NMB receptors on NMB-R transfected cells.

Figure 2 is a graph showing the ability of SS-14, SS-28 and various cyclo somatostatin octapeptide analogs to inhibit NMB-stimulated increases in [³H]inositol phosphates (IP) in NMB transfected cells.

Figure 3 is a graph showing the effect of increasing concentrations of cyclo SS-octa (i.e., Analog #1) on the dose-response curve for NMB-stimulated increases in [³H]IP in NMB-R transfected cells.

Figure 4 is a graph showing effect of a fixed concentration of cyclo SS-octa (i.e., Analog #1) on the dose-inhibition curve of NMB for inhibiting ¹²⁵I-[D-Tyr⁰]NMB binding to NMB-R transfected cells.

Certain somatostatin octapeptide analogs function as NMB-R receptor antagonists and have > 100-fold higher affinity for NMB-R than GRP-R. The most potent analog, cyclo SS-octa or H2-D-Nal-Cys-Tyr-D-Trp-Lys-Val-Cys-Nal-NH2, inhibited binding of ¹²⁵I-[D-Tyr⁰]NMB to NMB receptors on NMB-R transfected 3T3 cells (K_{d} 216 nM) and on glioblastoma C-6 cells (K_{d} 59 nM). This analog had a 100-fold lower affinity for GAP-R receptors on rat pancreatic acini.

Structure-function studies performed by synthesizing numerous structurally related SS-octapeptide analogs show that each of these analogs but not native SS-14 or SS-28 also inhibited binding to NMB receptors. [For Structures of SS-14 and SS-28 are well known in the art, see, e.g., Bachem California 1991-1992 Catalog, Torrance, CA.] The stereochemistry at positions 1, 2, 7, and 8, the hydrophobicity and ring size of the substitution in positions 1, 3, and 4 and the basicity of the group in position 5 all were important in determining NMB-R affinity. Each SS-octa analog did not increase [³H]IP in NMB-R transfected cells; however, each inhibited NMB-stimulated increases. The ability of each SS-octa analog to inhibit binding correlated closely with its ability to inhibit NMB-stimulated increases in [³H]IP.

The most potent analog, cyclo SS-octa, caused a parallel rightward shift of the NMB dose-response curve, the Schild plot was not significantly different from unity and the affinity was 230 nM. Furthermore, 0.5 *µ*M cyclo SS-octa caused a decrease in the NMB-R affinity and no change in the number of NMB-R binding sites demonstrating competitive antagonism. Cycle SS-octa did not inhibit Bn-stimulated amylase release from GRP receptor on pancreatic acini or increases in [³H]IP by endothelin in C-6 cells, nor binding of a number of different ligands to non-Bn-related receptors. Structure-function studies demonstrated that the SS-octa analogs also interacted with SS receptors and mu opioid receptors; however, there was no correlation between their affinities for these receptors and NMB-R, demonstrating these activities can be separated. The results demonstrate for the first time a class of antagonists with > 100-fold selectivity for NMB than GRP receptors.

It is believed that one skilled in the art can, based on the description herein, utilize the present invention to its fullest extent. The following specific embodiments are, therefore, to be construed as merely illustrative, and not limitative of the remainder of the disclosure in any way whatsoever.

### EXPERIMENTAL INFORMATION

### MATERIALS:

Rat glioblastoma C-6 cells were obtained from the American Type Culture Collection (Rockville, MD), Dulbecco's modified essential medium, fetal bovine serum, and Geneticin (aminoglycoside G-418) were from GIBCO (Waltham, MA), and cell culture flasks and 24-well plates were obtained from Costar Co. (Cambridge, MA).

Bovine serum albumin (fraction V) and HEPES were obtained from Boehringer Mannheim Biochemicals (Indianapolis, IN); soybean trypsin inhibitor, EGTA, and bacitracin were from Sigma Chemical Co. (St. Louis, MO); glutamine was from the Media Section, National Institutes of Health (Bethesda, MD); NMB, [Tyr⁴]Bn, bombesin, GRP, and endothelin 1 (ET-1) were from Peninsula Laboratories (Belmont, CA); Na¹²⁵I was from Amersham Co. (Arlington Heights, IL); *myo*-[2-³H]inositol (16-20 Ci/mmol) was from New England Nuclear (Boston, MA); somatostatin-14 (SS-14) and somatostatin-28 (SS-28) were from Bachem, (Torrence, CA); Dowex AG 1-X8 anion exchange resin (100-200 mesh, formate form) was from Bio-Rad (Richmond, CA); Hydro-Fluor scintillation fluid, methanol (absolute), and hydrochloric acid were from the J. T. Baker Chemical Co. (Phillipsburg, NJ).

### METHODS:

### Transfection and maintenance of cell lines

As described previously [Wada, E., et al. Neuron 6:421 (1991)], BALB 3T3 cells expressing a stably transfected rat NMB receptor (NMB-R transfected cells) were obtained using calcium phosphate precipitation of a full length NMB-preferring bombesin receptor clone generated from rat esophagus and subcloned into a modified version of the pCD2 plasmid. Cells were passaged every 3-4 days at confluence, using 0.1% trypsin in 1 mM EDTA. Rat glioblastoma C-6 tumor cells were maintained similarly and were passaged weekly at confluence. Both cell lines were cultured at 37°C in a 5% CO₂ atmosphere. Rat AR 42J pancreatic acinar cells were cultured in Dulbecco's modified Eagle's medium (DMEM) without antibiotics and supplemented with 10% (vol/vol) fetal calf serum. The incubation atmosphere consisted of 10% CO₂-90% humidified air at 37°C.

### Prenaration of rat pancreatic acini

Dispersed acini from guinea pig pancreas were prepared as described previously [Jensen, R. T. et al. *J. Biol. Chem.* 257:5554 (1982); and Peikin, S. R. et al. *Am. J. Physiol*. 235:G743 (1978)).

### Preparation of peptides

Peptides were synthesized on methybenzhydrylamine resin using standard solid phase procedures and cleaved with hydrogen fluoride/anisol mixtures. Peptides were cyclized in dilute 90% acetic acid solution by titration with I₂ and purified by gel filtration on Sephadex G-25 in 50% acetic acid and gradient elution on C18 silica using acetonitrile/0.1% trifluoroacetic acid buffers. The methods have been described in detail previously [Sasaki, Y. et al. *J. Med. Chem.* 30:1162 (1987); Stewart, J.M. et al. Solid Phase Peptide Synthesis, 2nd Ed., Pierce Chemical Co., Rockford, IL (1984); and Coy, D.H. et al. Tetrahedron, 44:835 (1988).] Homogeneity was assessed by thin layer chromatography, analytical HPLC, amino acid analysis and mass spectrometry and was determined to be > 96% for each peptide.

Below is a detailed description regarding the synthesis of Analog #1. Other peptides of the invention can be prepared by making appropriate modifications, within the ability of someone of ordinary skill in this field, of the synthetic methods disclosed herein.

### Step 1: Preparation of Boc-D-Nal-S-methylbenzyl-Cys-O-bromobenzyloxycarbonyl-Tyr-D-Trp-N-benzyloxycarbonyl-Lys-Val-S-methylbenzyl-Cys-Nal-benzhydrylamine resin

Benzhydrylamine-polystyrene resin (Advanced Chem Tech, Inc.) (1.2 g, 0.5 mmole) in the chloride ion form was placed in the reaction vessel of an Advanced Chem Tech peptide synthesizer programmed to perform the following reaction cycle: (a) methylene chloride; (b) 33% trifluoroacetic acid in methylene chloride (2 time for 1 and 25 min each); (c) methylene chloride; (d) ethanol; (e) methylene chloride; (f) 10% triethylamine in chloroform.

The neutralized resin was stirred with t-butyloxycarbonyl("Boc")-Nal and diisopropylcarbodiimide (1.5 mmole each) in methylene chloride for 1 hr and the resulting amino acid resin was then cycled through steps (a) to (g) in the above wash program. The following amino acids (1.5 mmole) were then coupled successively by the same procedure: Boc-S-methylbenzyl-Cys, Val, Boc-N-benzyloxycarbonyl-Lys, Boc-D-Trp, Boc-O-bromobenzyloxycarbonyl-Tyr, and Boc-S-methylbenzyl-Cys and Boc-D-Nal. After washing and drying, the completed resin weighed 1.78 g.

Step 2: Preparation of

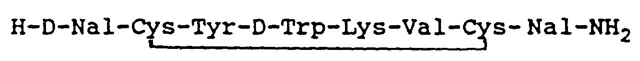
H-D-Nal-Cys-Tyr-D-Trp-Lys-Val-Cys-Nal-NH2


The peptide resin obtained from Step 1 (1.78 g, 0.5 mmole) was mixed with anisole (5 ml), dithiothreitol (100 mg) and anhydrous hydrogen fluoride (35 ml) at 0°C and stirred for 45 min. Excess hydrogen fluoride was evaporated rapidly under a stream of dry nitrogen and free peptide precipitated and washed with ether. The crude peptide was then dissolved in 500 ml of 90% acetic acid to which was added a concentrated solution of I₂/MeOH until a permanent brown color was observed. Excess I₂ was removed by addition of ascorbic acid and the solution evaporated to a small volume which was applied to a column (2.5 x 90 cm) of Sephadex G-25 which was eluted with 50% AcOH. Fractions containing a major component by UV absorption and thin layer chromatography ("TLC") were then pooled, evaporated to a small volume and applied to a column (1.5 x 70 cm) of Vydac octadecylsilane silica (10-15 *µ*), followed by elution with a linear gradient of acetonitrile in 0.1% trifluoroacetic acid in water. Fractions were examined by TLC and analytical high performance liquid chromatography ("HPLC") and pooled to give maximum purity.

Repeated lyophilization of the solution from water gave 151 mg of the product as a white, fluffy powder. The product was found to be homogeneous by HPLC and TLC. Amino acid analysis of an acid hydrolysate and FAB MS confirmed the composition of the octapeptide.

### Preparation of ¹²⁵I-[D-Tyr⁰]NMB

¹²⁵I-[D-Tyr⁰]NMB (2200 Ci/mmol) was prepared using Iodo-Gen as described recently [Benya, R. V. et al. *Mol*. *Pharmacol.* 42:1058 (1992)]. In brief, 0.4 *µ*g of Iodo-Gen was added to 8.0 *µ*g of [D-Tyr⁰]NMB with 2 mCi of Na¹²⁵ I in 20 *µ*l of 0.5 M KH₂PO₄ buffer (pH 7.4). After incubation at 22°C for 6 min, 300 *µ*l of 1.5 M dithiothreitol were added and the reaction mixture was incubated at 80°C for 60 min. Free ¹²⁵I was separated by applying the reaction mixture to a Sep-Pak cartridge (Waters Associates, Milford, MA), which had been prepared by washing with 5 ml of methanol, 5 ml of 0.1% trifluoroacetic acid, and 5 ml of water. Free ¹²⁵I was eluted with 200-*µ*l sequential elutions (10 times) of 60% acetonitrile/0.1% trifluoroacetic acid. Radiolabeled peptide was separated from unlabeled peptide by combining the three elutions with the highest radioactivity and applying them to a reverse phase high performance liquid chromatograph (Waters Associates model 204, with a Rheodyne injector), using a 0.46- x 25-cm *µ* BondaPak column. The column was eluted with a linear gradient of acetonitrile and 0.1% trifluoroacetic acid (v/v) from 16 to 64% acetonitrile in 60 min, with a flow rate of 1.0 ml/min. ¹²⁵I-[D-Tyr⁰]NMB was stored with 1% (w/v) BSA at -20°C and was stable for at least 6 weeks.

### Binding of ¹²⁵I-[D-Tyr⁰]NMB to C-6 glioblastoma and NMB-R transfected cells

Binding studies using rat glioblastoma C-6 or NMB-R transfected cells were performed as described previously [Benya, R. V. et al. *Mol. Pharmacol.* 42:1058 (1992); and Wang, L-H. et al. *Biochem*. *J.* 286:641 (1992)] by suspending disaggregated cells in binding buffer, which was composed of standing buffer (130 nM NaCl, 7.7 mM KCl, 1.0 mM EGTA, 0.02% soybean trypsin inhibitor) additionally containing 50 mM HEPES, 1 mM MgCl₂, 1.5 mM CaCl₂, 2.2 mM KH₂PO₄, 0.015% glutamine, and 0.2% BSA (w\v) (pH 7.4). Incubations contained 75 pM ¹²⁵I-[D-Tyr⁰]NMB and 15 x 10⁶ C-6 cells/ml or 2 x 10⁶ NMB-R transfected cells/ml, for 60 min at 22°C. Nonsaturable binding of ¹²⁵I-[D-Tyr⁰]NMB was te amount of radioactivity associated with C-6 cells or NMB-R transfected cells when the incubation mixture contained in *µ*M NMB. Nonsaturable binding was < 15% of total binding in all experiments; all values are reported herein as saturable binding (i.e., total minus nonsaturable binding).

Binding of ¹²⁵I-labeled [Tyr⁴]bombesin to acini. ¹²⁵I-[Tyr⁴]bombesin (2000 Ci/mmol) was prepared using the modification [Von Screnck et al. *Am. J. Physiol*. 256:G747 (1989)] of the method as described previously [Jensen, R. T. et al. *Proc*. *Natl*. *Acad*. *Sci*. *USA* 75:6139 (1978)]. ¹²⁵I-[Tyr⁴]bombesin was separated from ¹²⁵I using a Sep-Pak cartridge and separated from unlabeled peptide by reverse-phase high pressure liquid chromatography on a column (0.46 x 25 cm) of *µ*Bondapak C₁₈. The column was eluted isocratically with acetonitrile (22.5%) and triethylammonium phosphate (0.25 M, pH 3.5) (77.5%) at a flow rate of 1 ml/min. Incubations contained 50 pM ¹²⁵I-[Tyr⁴]bombesin and were for 60 min at 37°C with pancreatic acini. Nonsaturable binding of ¹²⁵I-[Tyr⁴]bombesin was the amount of radioactivity associated with the acini when the incubate contained 50 pM ¹²⁵I-[Tyr⁴]bombesin plus 1 *µ*M bombesin. All values shown are for saturable binding, i.e., binding measured with ¹²⁵I-[Tyr⁴]bombesin alone (total) minus binding measured in the presence of 1 *µ*M unlabeled bombesin (nonsaturable binding). Nonsaturable binding was < 10% of total binding in all experiments.

### Membrane receptor assays

Membranes were prepared from rat olfactory bulb (NMB membrane receptor assay), AR 42J cells (GRP and somatostatin membrane receptor assays), guinea pig cerebral (N₁, histamine H₁, and sigma opioid membrane receptor assays), rat pancreas (CCK_{A} receptor assay), rat cerebral cortex (CCK_{B}, PYY, neurotensin, ∝₁-adrenergic, ∝₂-adrenergic, muscarinic cholinergic, neural benzodiazepine, peripheral benzodiazepine, adenosine, calcium channel, and N-methyl-D-asparate membrane receptor assays), A10 smooth muscle cells (Et_{A} membrane receptor assay), rat forebrain (TRH, mu and delta opioid membrane assays), rat corpus striatum (dopamine₁ and dopamine₂ membrane receptor assays), and rat frontal cortex (serotonin₂ membrane receptor assay). Membranes were prepared using a Polytron (setting 6, 15 sec) in ice-cold 50 mM Tris-HCl unless otherwise specified below and centrifuged twice at 39,000 x g/10 min) with an intermediate resuspension in fresh buffer. For the NMB and GRP membrane receptor assay, final pellets were resuspended in 50 mM Tris-HCl containing 0.1 mg/ml bacitracin, and 0.1% BSA and for the somatostatin receptor assay in 10 mM Tris-HCl. For the NMB and GRP membrane binding assay 50 pM ¹²⁵I-NMB (or 50 pM ¹²⁵I-[Tyr⁴]Bn) was used with a 30 min incubation at 4°C, whereas for the somatostatin assay the incubation was 25 min at 30°C with 50 pM ¹²⁵I-[Tyr¹]somatostatin in 50 mM HEPES (pH 7.4) with 0.1% BSA, 5 mM MgCl2, bacitracin (0.02 mg/ml), trasylol (200 KIU/ml) and phenylmethylsulfonyl fluoride (PMSF) (0.02 mg/ml). Incubations were terminated by rapid filtration through GF/B filters presoaked in 0.1% polyethyleneimine (NMB and GRP receptor assays). Each filter was washed three times with 5 ml aliquots of ice-cold buffer. The ligands used for the various membrane binding assays were [³H]substance P (NK₁ receptor), [¹²⁵I]endothelin-1 (endothelin_{A} receptor), [¹²⁵I]CCK-8 (CCK_{A} and CCK_{B} receptors), [¹²⁵I]PYY (PYY receptor), [³H]neurotensin (neurotensin receptor), [³H]bradykinin (bradykinin₂ receptor), [³H]3-meHisTRH (TRH receptor), [³H]prazosin (ω₁ adrenergic receptor), [³H]clonidine (ω₂ adrenergic receptor), [³H]dihydroalprenolol (β₁ adrenergic receptor), [³H]QNB (muscarinic cholinergic receptor), [³H]RO15-1788 (benzodiazepine-neural receptor), [³H]RO5-4864 (benzodiazepine-peripheral receptor), [³H]SCh 23390 (dopamine₁ receptor), [³H]spiperone (dopamine₂ receptor), [³H]ketanserin (serotonin₂ receptor), [³H]pyrilamine (histamine H₁ receptor), [³H]cyclohexyladenosine (adenosine₁ receptor), [³H]MK-801 (N-methyl-D-aspartate receptor), [³H]pentazocine (sigma opioid receptor), [³H]DAGO (mu opioid receptor) and [³H]DPDPE (dela opioid receptor).

### Measurement of phosphoinositides

Total phosphoinositides in C-6 cells and in NMB-R transfected cells were determined as described previously, with minor modifications [Benya, R. V. et al. *Mol. Pharmacol.* 42:1058 (1992); and Wang, L-H. et al. *Biochem*. *J.* 286:641 (1992)]. Cells were grown to confluence in 24-well plates and then loaded with 100 *µ*Ci/ml *myo*-[2-³H]inositol in Dulbecco's modified essential medium with 2% fetal bovine serum at 37°C for 48 hrs. Cells were washed and incubated in phosphoinositide buffer (standard buffer additionally containing 10 mM LiCl, 20 mM HEPES, 2 mM CaCl₂, 2% BSA, and 1.2 mM MgSO₄) for 15 min and then for 60 min at 37°C with agonists at various concentrations or with 3 nM NMB (a half-maximal effective concentration) and possible antagonists at different concentrations. Reactions were halted using ice-cold 1% HCl in methanol, and the inositol phosphates (IP) were isolated as described previously [Bologna, M. et al. *Cancer* 63:1714 (1989); and Endo, T. et al. *J. Endocrinol.* 131:313 (1991)]. Briefly, after loading of the anion exchange column, free [³H]glycerophosphorylinositol was removed by washing with 5 mM disodium tetraborate in 60 mM sodium formate. Total [³H]inositol phosphates were then eluted using 100 mM formic acid in 1.0 M ammonium formate as described previously [Benya, R. V. et al. *Mol. Pharmacol*. 42:1058 (1992); and Wang, L-H. et al. *Biochem*. *J.* 286:641 (1992)].

The words Sephadex, Sep-Pak and BondaPak used herein are Trade Marks.

### RESULTS:

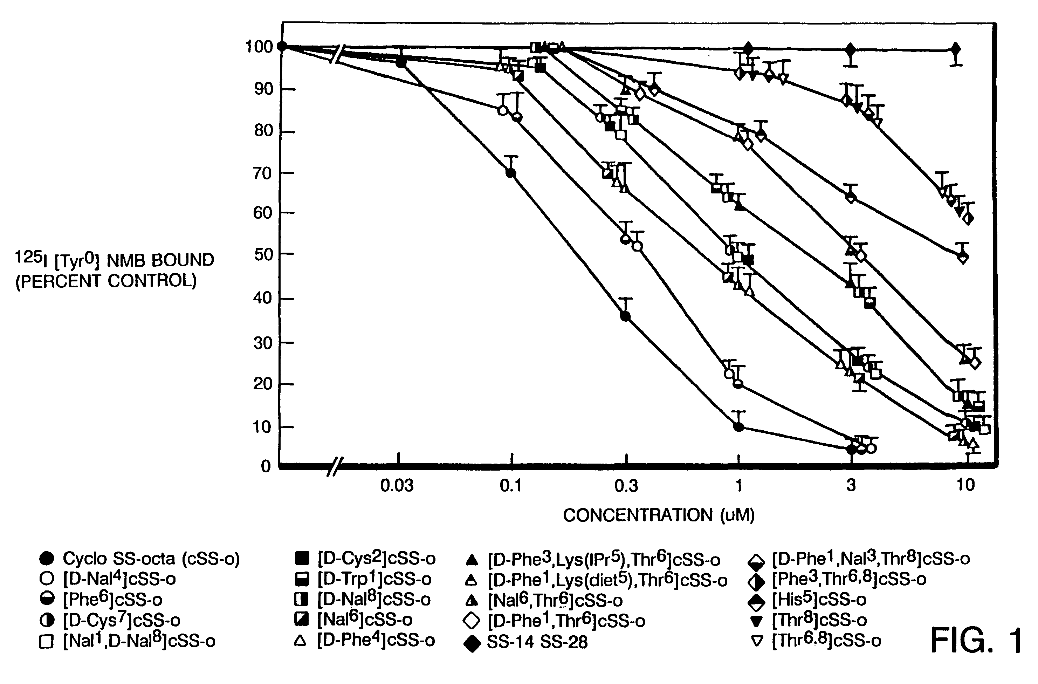

To investigate the ability of SS-14, SS-28 and the various cycle SS-octa analogs to interact with NMB and GRP receptors, the ability of each to inhibit binding of either ¹²⁵I-[D-Tyr⁰]NMB to NMB-R transfected cells (Fig. 1) or ¹²⁵I-[D-Tyr⁴]Bn to GRP receptors on rat pancreatic acini was determined. At 10 *µ*M, SS-14 and SS-28 caused no inhibition of binding of ¹²⁵I-[Tyr⁰]NMB to NMB-R transfected cells; however, each of the cyclo SS-octapeptide analogs caused significant inhibition of binding of ¹²⁵I-[Tyr⁰]NMB to these cells (Fig. 1). Cyclo SS-octa (#1; Table 1) was the most potent causing detectible inhibition of binding of ¹²⁵I-[Tyr⁰]NMB at 0.1 µM, half-maximal inhibition at 216 nM and complete inhibition at 3 *µ*M (Fig. 1; Table 1). Cyclo SS-octa was 2-fold more potent than [Phe⁶]-cycle SS-octa (#13; Table 1); and [D-Nal⁴]-cycle SS-octa (#17; Table 1) (K₁, 400 nM; Table 1) which were equipotent; 4-fold more potent than [Nal⁶,Thr⁸]-, [Nal⁶]- and [D-Phe¹]-cyclo SS-octa (Fig. 1) (#2-4; Table 1; K₁ 700-800 nM); 6-fold more potent than (Nal¹,D-Nal⁸]-cyclo SS-octa, [D-Cys²]- and [D-Cys⁷]-cyclo SS-octa (Fig. 1) (#9-11; Table 1; K₁ 1-1.2 *µ*M); 9-fold more potent than [D-Nal⁸]-, [D-Trp¹]- and [D-Phe¹, Lys(iPr)⁵, Thr⁶]-cyclo SS-octa (#8, 12 and 15; Table 1; K₁ 1.4-2.3 *µ*M); 18-fold more potent than [D-Phe¹, Lys(diEt)⁵, Thr⁶] cyclo SS-octa (#7 and 16; Table 1; K₁ 3.9-4.4 *µ*M); 45-fold more potent than [His⁵]-cyclo SS-octa (#14; Table 1; K₁ 9.9 *µ*M) and 69-fold more potent than [Thr⁸]-, [Thr^{6,8}]-, [Phe³, Thr^{6,8}]-, and [D-Phe¹,Nal³,Thr⁸]-cycle SS-octa (#5, 6, 18 and 19; Table 1; K₁ 14-19 *µ*M). In contrast but similar to SS-14 and SS-28, 13 of the cyclo SS-octapeptides caused no inhibition of ¹²⁵I-[Tyr⁴]Bn binding to GRP receptors on rat pancreatic acini, and the remaining six analogs had very low affinity for this receptor with each having an affinity > 15 *µ*M (Table 1). The three most potent analogs, cyclo SS-octa, [Phe⁶]-cycle SS-octa and [D-Nal⁴]-cyclo SS-octa (#1, 13 and 17; Table 1) had 84, > 100 and > 100-fold greater affinity for the NMB than the GRP receptors (Table 1).

**Table 1:**

| Affinity of SS-14, SS-28 or various ss-octapeptide analogs for NMB receptors on C-6 cells or transfected BALB 3T3 cells or GRP receptors on rat pancreatic acini. | | | | | |
|---|---|---|---|---|---|
| NMB Receptor | | | | GRP Receptor | |
| | C-6 Cells | Transfected Cells | | Rat Pancreatic Acini | |
| Analog # | Ki(nM) ¹²³I-NMB Binding | IC₅₀ (nM) NMB-stimulated [³H]IP | Ki (nM) ¹²³I-NMB Binding | IC₅₀ (nM) Amylase Release | Ki (nM) ¹²³I[Tyr⁴]BnBinding |
| 1 | 59 ± 9 | 885 ± 98 | 216 ± 36 | - - | 18264 ± 2110 |
| 2 | 226 ± 36 | 6673 ± 435 | 772 ± 94 | - - | 16291 ± 3818 |
| 3 | 997 ± 76 | 2880 ± 188 | 697 ± 64 | - - | - - |
| 4 | 848 ± 191 | 3757 ± 568 | 818 ± 68 | - - | 21947 ± 4265 |
| 5 | 3792 ± 1084 | - - | 14766 ± 2651 | - - | - - |
| 6 | 8286 ± 2427 | - - | 16398 ± 4455 | - - | - - |
| 7 | 1452 ± 78 | 36235 ± 4974 | 4362 ± 328 | - - | 30981 ± 4653 |
| 8 | 670 ± 73 | 5187 ± 987 | 1924 ± 201 | - - | - - |
| 9 | 1159 ± 214 | 4413 ± 451 | 1156 ± 229 | - - | - - |
| 10 | 1147 ± 518 | 3992 ± 781 | 960 ± 109 | - - | - - |
| 11 | 1740 ± 345 | 1427 ± 119 | 1077 ± 199 | - - | - - |
| 12 | 1778 ± 109 | 4688 ± 927 | 1411 ± 127 | - - | - - |
| 13 | 213 ± 13 | 1173 ± 114 | 397 ± 72 | - - | - - |
| 14 | 4944 ± 930 | 11865 ± 1835 | 9863 ± 1294 | - - | - - |
| 15 | 1142 ± 105 | 9897 ± 2312 | 2328 ± 397 | - - | 38127 ± 21549 |
| 16 | 1089 ± 38 | 7212 ± 2795 | 3951 ± 509 | - - | - - |
| 17 | 313 ± 33 | 1779 ± 295 | 399 ± 68 | - - | - - |
| 18 | 8322 ± 957 | > 10 *µ*M | 19816 ± 4235 | - - | 61637 ± 21512 |
| 19 | 8485 ± 1165 | > 10 *µ*M | 14341 ± 1819 | - - | - - |
| SS-14 | - - | - - | - - | - - | - - |
| SS-15 | - - | - - | - - | - - | - - |
| - - = no agonist or antagonist activity at concentrations up to 10 µM. IC₅₀ = concentration causing half-maximal inhibition of the indicated agonist. Ki = affinity of the indicated peptide for the indicated receptor calculated by the method of Cheng, Y. C. et al. Biochem. Pharmacol. 22:2099 (1973). | | | | | |

To determine whether SS-14, SS-28 or the various cyclo SS-octapeptide analogs functioned as agonists or antagonists at the Bn receptor subtypes, their ability at 10 *µ*M to stimulate increases in [³H]IP in NMB-R transfected cells or stimulate amylase release or inhibit Bn-stimulated amylase release from rat pancreatic acini possessing GRP receptors was assessed (Table 2). Neither SS-14, SS-28 nor any of the 19 SS-octapeptide analogs at a concentration of 10 *µ*M had agonist activity and stimulated increases in [³H]IP in NMB-R transfected cells or amylase release from rat pancreatic acini which have GRP receptors (Table 2). Similarly, none of these peptides at this concentration altered the increase in amylase release caused by 0.3 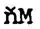 Bn in rat pancreatic acini (Table 2). Whereas SS-14, SS-28 and 3 cyclo SS-octapeptide analogs (#5, 6 and 18; Table 2) had no effect on the 14-fold increase in [³H]IP caused by 3 nM NMB in NMB-R transfected cells, 16 of the cyclo SS-octapeptide analogs caused some inhibition (Table 2). Five analogs at 10 *µ*M (#1, 10, 11, 13 and 17; Table 2) completely inhibited the NMB-stimulated increase in [³H]IP.

**Table 2:**

| Affinity of SS-14, SS-28 and related octapeptide analogs to alter NMB-stimulated increases and [³H]lP in NMB-R transfected cells or amylase release in rat pancreatic acini. | | | | |
|---|---|---|---|---|
| | NMB-R Transfected Cells [³H]IP (dpm x 10³) | | Rat Pancreatic Acini Amylase Release (% total) | |
| Analog # | Alone (10 *µ* M) | Plus NMB (3 nM) | Alone (10*µ*M) | Plus Bn(0.3 nM) |
| | 13 ± 3 | 186 ± 58 | 4 ± 1 | 15 ± 1 |
| 1 | 12 ± 2 | 8 ± 4* | 5 ± 1 | 15 ± 2 |
| 2 | 11 ± 2 | 39 ± 7* | 5 ± 1 | 13 ± 1 |
| 3 | 11 ± 1 | 65 ± 15* | 3 ± 1 | 15 ± 1 |
| 4 | 13 ± 1 | 33 ± 4* | 4 ± 2 | 14 ± 1 |
| 5 | 11 ± 2 | 179 ± 13 | 3 ± 1 | 12 ± 1 |
| 6 | 11 ± 2 | 188 ± 30 | 3 ± 1 | 14 ± 2 |
| 7 | 15 ± 1 | 99 ± 24* | 3 ± 1 | 16 ± 1 |
| 8 | 10 ± 3 | 67 ± 13* | 2 ± 1 | 14 ± 1 |
| 9 | 8 ± 2 | 28 ± 6* | 3 ± 1 | 16 ± 1 |
| 10 | 10 ± 3 | 4 ± 2* | 3 ± 1 | 16 ± 1 |
| 11 | 10 ± 1 | 7 ± 4* | 2 ± 1 | 14 ± 1 |
| 12 | 9 ± 2 | 43 ± 2 | 3 ± 1 | 13 ± 1 |
| 13 | 10 ± 1 | 9 ± 4* | 3 ± 1 | 14 ± 1 |
| 14 | 8 ± 1 | 97 ± 7* | 2 ± 1 | 13 ± 1 |
| 15 | 11 ± 1 | 67 ± 7* | 4 ± 2 | 13 ± 2 |
| 16 | 11 ± 2 | 73 ± 15* | 5 ± 1 | 12 ± 1 |
| 17 | 12 ± 4 | 15 ± 2* | 4 ± 2 | 19 ± 1 |
| 18 | 11 ± 1 | 143 ± 11 | 3 ± 1 | 14 ± 1 |
| 19 | 10 ± 1 | 119 ± 4 | 3 ± 1 | 14 ± 2 |
| SS-14 | 14 ± 1 | 182 ± 24 | 3 ± 1 | 12 ± 1 |
| SS-28 | 12 ± 1 | 177 ± 13 | 4 ± 1 | 10 ± 1 |

| | | | | |
|---|---|---|---|---|
| * = p < 0.05 compared to value with no somatostatm analog added. Rat pancreatic acini or [³H]myo-[2-³H]inositol-loaded NMB-R tansfected cells were incubated either with no additives, the somatostatin octapeptide analog, Bn, or NMB, or a combination for 30 min at 3TC- Amylase release from pancreatic aciru was expressed at the indicated percent of the total cellular amylase released dunng the incubation. [³H]IP is expressed in dpm's. To test for inhibitory effects. the effeet of somatostatin analog (10 *µ*M) was determined on 0.3 nM Bn-stimulated amylase release or a 4 nM NMB-stimulated increase in [³H]IP which are each half-maximally effective agonist concentrations. Results are means ± ISEM from at least 4 separate experiments and in each expenment, each value was determined in duplicate. | | | | |

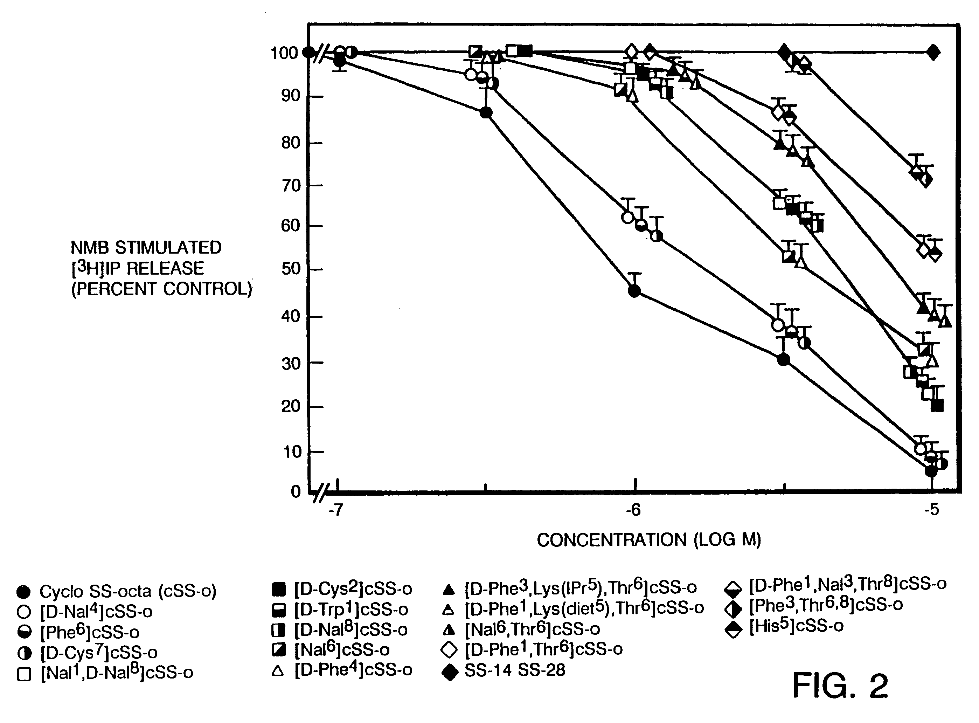

To determine the relative abilities of the SS-octapeptide analogs to inhibit NMB-stimulated increases in [³H]IP in NMB-R trans, transfected cells, dose-inhibition curves were determined for each analog (Fig. 2). Cyclo SS-octa (#1; Table 1) was the most potent, causing detectible inhibition at 0.3 *µ*M, half-maximal inhibition at 885 nM and complete inhibition at 10 *µ*M (Fig. 2). The relative potencies were: cyclo SS-octa (#1, IC₅₀ 885 nM) > [D-Cys⁷]-, [Phe⁶]-, [D-Nal⁴]-cyclo SS-octa (#11, 13 and 17; Table 1); IC₅₀ 1.2-1.8 *µ*M) > [Nal⁶,Thr⁸]-, [Nal⁶]-, [D-Phe¹]-cyclo SS-octa (#2-4; Table 1); IC₅₀ 3-6.6 *µ*M) > [D-Nal⁸], [Nal¹,D-Nal⁸]-, [D-Cys²)-, [D-Trp¹]-cyclo SS-octa (#8-10, 12; Table 1; IC₅₀ 4.4-5.2 *µ*M) > [D-Phe¹,Lys(iPr)⁵,Thr⁶]-, [D-Phe¹,Lys(diEt)⁵,[Thr⁶]-cyclo SS-octa (#15 and 16; Table 1; IC₅₀ 7.2-9.8 *µ*M) > [His⁵]-cyclo-SS-octa (#14; Table 1, IC⁵⁰ 11.8 *µ*M) > [D-Phe¹, Thr⁶]-, [D-Phe¹,Nal³,Thr⁸]-, [Phe³,Thr^{6,8}]-cyclo-SS-octa (#7, 18 and 19; Table 1, IC₅₀ > 10 *µ*M) > SS-14, SS-28, [Thr⁸]-, [Thr^{6,8}]-cyclo-SS-octa (#5 and 6, no activity at 10 *µ*M). In general, there was a close agreement between the relative abilities of the different SS-octapeptide analogs to occupy the NMB-receptor and inhibit binding of ¹²⁵I-[D-Tyr⁰]NMB to NMB-R transfected cells and their abilities to inhibit NMB-stimulated increases in [³H]IP in thase cells (Figs. 1, 2; Table 1).

The 18 cyclo SS-octapeptide analogs of cyclo SS-octa were made to explore the importance of the different amino acid substitutions in cyclo SS-octa (#1; Tables 1,2) in determining its ability to function as a NMB receptor antagonist. Analogs 8-11 (Tables 1, 2) explored the importance of stereochemistry at positions 1, 2, 7, 8 of cyclo SS-octa. Changing Cys¹ or Cys⁷ to a D-Cys had an equal effect in decreasing affinity 5-fold for both substitutions (compare #1, 10 and 11; Table 1). Similarly, insertion of a D-Nal in position 8 caused a 6-fold decrease in affinity (compare #1 and 8; Table 1) and the further addition of Nal¹ for D-Nal¹ did not change affinity further (compare #1, 8 and 9; Table 1). The importance of the hydrophobicity and ring size of the substituted amino acid was explored for position 1 (analog #4 and 12; Table 1), position 3 (#18 and 19; Table 1) and position 4 (#17; Table 1). The insertion of a less hydrophobic group with a different ring size, D-Phe¹ or D-Trp¹, had only a moderate effect, decreasing potency 4 to 7-fold (compare #1, 4 and 12; Table 1). In contrast, the insertion of more hydrophobic groups Nal³, D-Phe³ for Tyr³ had almost no effect on affinity (compare #5 and 6 with #18 and 19; Table 1) in that when added to an analog with a Thr⁸ replacement (#5; Table 1) no change in affinity occurred. Similarly, the insertion of the more hydrophobic group D-Nal for D-Trp in position 4 (compare #1 and 17; Table 1) had almost no effect on affinity. The importance of the Val substitution in position 6 of cyclo SS-octa was examined in analogs (#3 and 13; Table 1). Substitution of either Phe⁶ (#13; Table 1) or Nal⁶ (#3; Table 1) caused only a minimal (2 to 3-fold) decrease in affinity. The insertion of Thr⁸ in position similar to that used in some high affinity SS or mu receptor agonists [Maurer, R. et al. *Proc. Natl. Acad*. *sci. USA* 79:4815 (1982); Pelton, J. T. et al. *Proc*. *Natl. Acad. Sci.* 82:236 (1985); Gulya, K., et al. *Life Sci*. 38:2225 (1986); and Walker, J. M. et al. *Peptides* 8:869 (1987)], caused a dramatic (80-fold) decrease in NMB receptor affinity (compare #1 and 5; Table 1), whereas insertion of a Nal for Val⁶ compensated for the Thr⁸ substitution and resulted in only a 4-fold decrease (compare #1, 2 and 5; Table 1). The substitution of a less basic group His⁵ in position 5 for Lys caused a marked 50-fold decrease in affinity (compare #1 and 14; Table 1). [His⁵]-cyclo-SS-octa is not within the scope of this invention. Altering the availability of the primary amino group on Lys³ by formation of diethyl Lys³ or isopropyl Lys³ caused little change in affinity for the NMB receptor (compare #7, 15 and 16; Table 1).

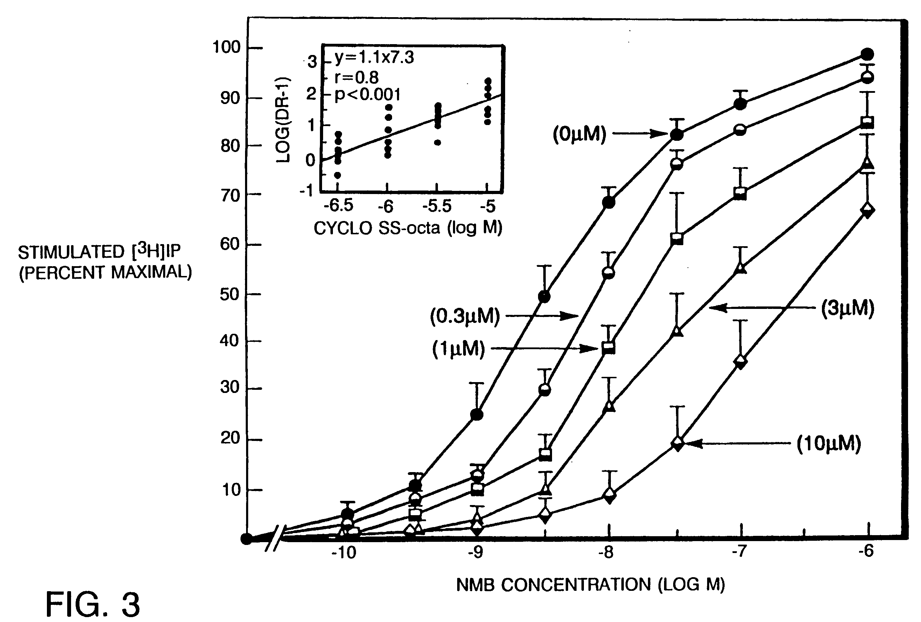

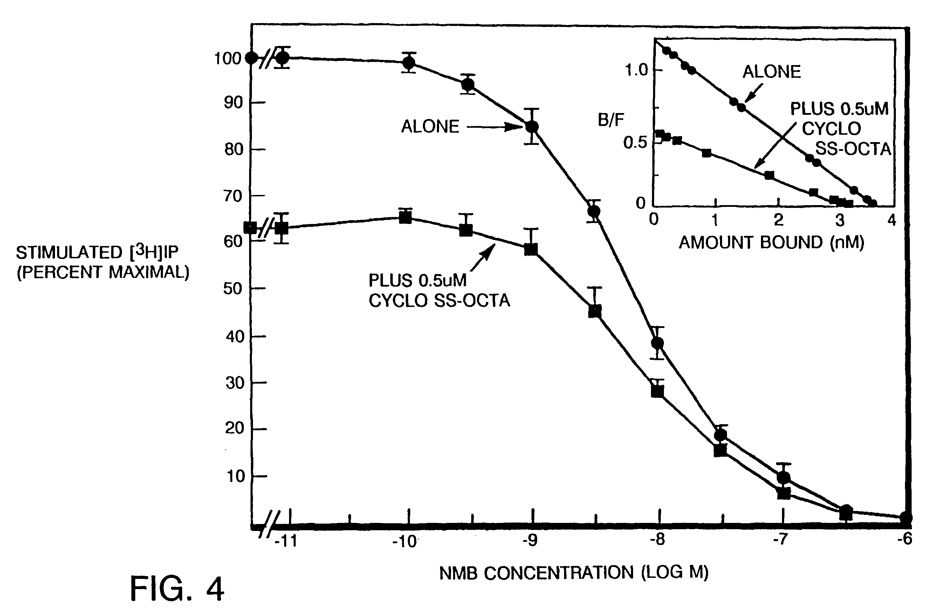

To investigate further the inhibitory action of the most potent analog, cyclo SS-octa (#1; Tables 1,2), its ability to affect the dose-response curve of NMB-stimulated increases in [³H]IP in NMB-R transfected cells was determined (Fig. 3) or the dose-inhibition curve of NMB for binding of ¹²⁵I-[D-Tyr⁰]NMB (Fig. 4) to these cells. The addition of I *µ*M, 3 *µ*M or 10 *µ*M cyclo SS-octa caused a parallel rightward shift in the dose-response curve for NMB-stimulated increases in [³H]IP with no change in the maximal increase if sufficiently high concentrations of NMB were used (Fig. 3). The magnitude of the rightward shift was proportional to the concentration of cyclo SS-octa and the concentration of NMB used (Fig. 3). Plotting these data in the form of Schild [Schild:H. O. *Br. J. Pharmacol*. 4:277 (1949)] (Fig. 3, insert) gave a regression equation of y = 1.1(+0.2)x ±7.3 with a correlation coefficient of 0.8 (p < 0.001) and the slope was not significantly different from unity. Calculation of the affinity of cyclo SS-octa for the NMB receptor from these data gave an affinity of 231±43 nM. Analysis of the ability of 0.5 *µ*M cyclo SS-octa to affect the dose-response curve of the ability of NMB to inhibit binding of ¹²⁵I-[D-Tyr⁰]NMB to NMB-R transfected cells using a nonlinear least-squares curve fitting program [Munson, P. J. et al. *Ann. Biochem.* 107:220 (1980)] demonstrated that cyclo SS-octa was functioning as a competitive antagonist (Fig. 4). Specifically, 0.5 *µ*M cyclo SS-octa caused a decrease in the affinity of NMB for NMB receptors (without cyclo SS-octa K_{d} 3.1±0.2 and with 0.5 *µ*M cyclo SS-octa present, K_{d}, 6.0±0.5, p < 0.01). In contrast, there was no change in the total number of NMB binding sites in the presence of 0.5 *µ*M cyclo SS-octa (without cyclo SS-octa = 7.6±0.5 pm/mg protein and with 0.5 *µ* M cyclo SS-octa = 6.6±0.5 pm/mg protein).

To investigate the specificity of the inhibitory effects of the cyclo SS-octa analogs, the ability of a number of these analogs to inhibit binding to a number of different ligands for different receptors was determined as well as their ability to interact with NMB receptors on C-6 glioblastoma cells and alter biological responses in these cells. C-6 glioblastoma cells have been shown to possess NMB receptors [Lin, W. W. et al. *J. Neurosci*. 12:1077 (1992)] and therefore the ability of SS-octapeptide analogs to interact with native receptors on these cells was compared to their ability to interact with NMB receptors on NMB-R transfected cells. As demonstrated in Table 1, SS-14 and SS-28 did not inhibit binding of ¹²⁵I-[D-Tyr⁰]NMB to C-6 cells, and cyclo SS-octa was the most potent SS-octapeptide analog having an affinity of 59±9 nM. The affinities of the other 18 SS octapeptide analogs was, in general, in good agreement with that seen for the NMB receptor on NMB-R transfected cells (Table 1). The specificity of the inhibitory action of two of the most potent analogs, cyclo SS octa (#1; Table 1) and [Nal⁶,Thr⁸)-cyclo SS-octa (#2; Table 1) was demonstrated on C-6 glioblastoma cells because each of these SS-octapeptide analogs inhibited NMB-stimulated increases in [³H]IP in these cells, but had no effect on endothelin-1-simulated increases in [³H]IP (Table 3). Furthermore, the most potent SS-octapeptide analog, cyclo SS-octa (#1; Tables 1, 2), at 1 *µ*M, a concentration that inhibited binding to NMB receptors on rat olfactory bulb membranes by > 95%, did not inhibit binding to CCK_{A}, CCK_{B}, endothelin_{A}, PYY, bradykinin₁, TRH, α₁- or α₂- adrenergic, β₁-adrenergic, muscarinic cholinergic, benzodiazepine-neural or peripheral, dopamine₂, histamine H₁, adenosine₁, sigma or delta opioid, N-methyl D-aspartate receptors on plasma membranes on various tissues determined as described in METHODS.

**Table 3:**

| Ability of two cyclic somatostatin octapeptide analogs to inhibit NMB- and endothelin-stimulated increases in inositol phosphates in C-6 glioblastoma cells | | | |
|---|---|---|---|
| | | [³H]IP (dpm x 10³) | |
| Peptide Added | Alone | Nal⁶,Thr⁸]-cyclo SS-octa (10 *µ*M) | Cyclo SS-octa (10 *µ*M) |
| None | 7.2±1.0 | 7.3±1.2 | 7.4 ± 1.3 |
| ET-1 (0.1 nM) | 11.0±2.1 | 11.2±1.9 | 10.9 ± 1.8 |
| NMB (10 nM) | 21.0 ± 1.8 | 14.3 ± 4.2* | 7.5 ± 1.0** |
| Significantly different (* = p<0.05), (** = p<0.01) compared to value without SS-octapeptide analog added. C-6 glioblastoma cells (50,000 cells/well) were incubated with myo-[2-³H]inositol for two days, washed and then incubated with or without the indicateet peptides for 60 minutes at 37°C in phosphoinositide buffer containing 10 mM LiCl as described in METHODS. [³H]IP was measured using Dowex AGl-X8 anion exchange chromatography as described in METHODS. Results are means ± SEM from three experiments and in each experiment each value was determined in duplicate. | | | |

Cyclo SS-octa did inhibit binding of [³H]DAGO to mu opioid receptors on rat forebrain membranes with an affinity of 430±130 nM (Table 4) and inhibited binding of ¹²⁵I-CCK-8 to CCK_{A} receptors on rat pancreatic membranes with an affinity of 5537±7 nM. In previous studies various SS analogs have been reported to have high affinity for mu opioid receptors as well as somatostatin receptors [Maurer, R. et al. *Proc. Natl. Acad. Sci. USA* 79:4815 (1982); Pelton, J. T. et al. *Proc. Natl. Acad. Sci*. 82:236 (1985); Gulya, K., et al. *Life Sci.* 38:2225 (1986); and Walker, J. M. et al. *Peptides* 8:869 (1987)]. To compare the ability of SS-14 and SS-28 and the various SS octapeptide analogs to interact with both subtypes of Bn receptors, somatostatin and mu opioid receptors in membranes from the same species, the ability of each of these peptides to inhibit binding of ¹²⁵I-[Tyr¹¹]SS-14 or ¹²⁵I-[Tyr⁴]Bn to cell membranes from the rat pancreatic acinar cell tumor, AR 42J cells, binding of ¹²⁵I-NMB to NMB receptors on rat olfactory bulb membranes, and binding of [³H]DAGO to rat forebrain membranes was determined (Table 4). Neither NMB nor GRP at concentrations up to 10 *µ* M inhibited binding of ¹²⁵I-[Tyr¹¹]SS-14 to somatostatin receptors on AR 42J cells or mu opioid receptors on rat forebrain membranes, and neither SS-14 nor SS-28 at concentrations up to 10 *µ*M inhibited binding to GRP receptors on AR 42J cell membranes, NMB receptors on rat olfactory bulb membranes or mu opioid receptors on rat forebrain membranes (Table 4). There was no correlation between the affinities of the various SS-octapeptide analogs for NMB receptors on rat olfactory bulb membranes and their affinities for somatostatin receptors on AR 42J cells (r = 0.1, p > 0.8), their affinities for GRP receptors on rat pancreatic membranes (r = 0.01, p > 0.5) or their affinities for mu opioid receptors on membranes from rat forebrain (r = 0.1, p > 0.7) (Table 4). For example, the cyclo SS analogs cyclo SS-octa (#1; Table 1), [Nal⁶,Thr⁸]-cyclo SS-octa (#2; Table 1), [D-Phe¹]-cyclo SS-octa (#4; Table 1), [D-Cys⁷]-cyclo SS-octa (#11; Table 1) and [Phe⁶]-cyclo SS-octa varied less than 5-fold in potency for NMB receptors yet varied 800-fold for affinity for somatostatin receptors and greater than 5000-fold for mu opioid receptors (Table 4). Whereas most of the SS-octa analogs had significantly higher affinity for somatostatin receptors than NMB receptors one analog, [His⁵]-cyclo SS-octa had a 3-fold higher affinity for NMB receptors. The most potent NMB receptor antagonist cyclo SS octa (#1; Table 4) had a 10-fold greater affinity for NMB receptors than mu opioid receptors and one analog (#11; Table 4) had > 50-fold higher affinity. These data demonstrate that the structural requirements of cyclo SS-octapeptide for high affinity NMB receptor occupation differ markedly from those required for high affinity somatostatin or mu opioid receptor occupation.

**Table 4:**

| Comparison of the affinity of NMB, GRP, SS-14, SS-28 and various SS octapeptide analogs for neuromedin B, GRP, somatostatin or mu opioid receptors on plasma membranes from AR 42J cells, rat olfactory bulb, or forebrain, respectively. | | | | |
|---|---|---|---|---|
| | AR 42J Cell Membranes | | Rat Olfactory | Rat Forebrain |
| Analog # | ¹²⁵I-[Tyr¹¹]SS-14 | ¹²⁵I-[Tyr⁴]Bn | ¹²⁵I-NMB | [³H]DAGO |
| NMB | >10,000 | 19 ± 1 | 1.1 ± 0.2 | >10,000 |
| GRP | >10,000 | 1.8 ± 0.1 | 297 ± 15 | >10,000 |
| SS-14 | 0.13 ± 0.01 | >50,000 | >40,000 | >10,000 |
| SS-28 | 0.40 ± 0.20 | >10,000 | >10,000 | >10,000 |
| 1 | 0.80 ± 0.50 | 2870 ± 520 | 43 ± 9 | 430 ± 130 |
| 2 | 0.50 ± 0.10 | 950 ± 70 | 85 ± 20 | 1.9 ± 0.7 |
| 4 | 0.24 ± 0.13 | 2000 ± 150 | 245 ± 130 | 650 ± 130 |
| 5 | 0.29 ± 0.03 | 3900 ± 1200 | 800 ± 200 | 2.0 ± 1.4 |
| 7 | 0.86 ± 0.23 | 750 ± 10 | 740 ± 160 | 200 |
| 8 | 3.9 ± 0.1 | 4100 ± 750 | 1480 ± 500 | 280 |
| 9 | 2.8 ± 0.7 | 1540 ± 180 | 590 ± 170 | 1160 |
| 10 | 91 ± 23 | 1500 ± 160 | 920 ± 350 | 1020 |
| 11 | 48 ± 3 | 7620 ± 1260 | 230 ± 70 | >10,000 |
| 12 | 3.1 ± 1.6 | 2100 ± 150 | 1180 ± 520 | >10,000 |
| 13 | 194 ± 27 | 2150 ± 200 | 270 ± 90 | 1640 |
| 14 | 1870 ± 30 | 3100 ± 700 | 470 ± 240 | 500 |
| 17 | 570 ± 180 | 3710 ± 200 | 850 ± 300 | 1570 |
| Membranes prepared from rat olfactory bulb, rat forebrain or AR 42J cells as described in METHODS were incubated with the indicated ligands as described in METHODS. Affinities were calculated by the method of Cheng, Y.C. et al. Biochem. Pharmacol. 22:2099 (1973). Results are means ± ISEM from at least three experiments. | | | | |

Further evidence that the various cyclo SS-octapeptide analogs were not altering NMB receptor affinity by occupying SS receptors was that no saturable binding of ¹²⁵I[Tyr¹¹]SS-14 was detected to glioblastoma C-6 cells or the NMB-R transfected cells (n=3). The ¹²⁵I-[Tyr¹¹]SS-14 used bound to dispersed guinea pig pancreatic acini, which has been shown to possess high affinity SS receptors [Esteve, J P et al Am J Physiol 247:G62 (1984)].

## Claims

1. Use of an octapeptide of the formula: 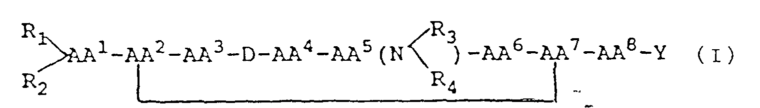 wherein
AA¹ is the D-isomer of an aromatic α-amino acid;
AA² is the L-isomer of Cys;
AA³ is Tyr;
AA⁴ is Trp;
AA⁵ is Lys;
AA⁶ is Leu, Ile, Nle, Val, Nal, Trp, Me-Trp, Bpa, F₅Phe, Phe, or 4-X-Phe in which X is halogen, NO₂, CH₃, or OH;
AA⁷ is the L-isomer of Cys;
AA⁸ is the L-isomer of an aromatic α-amino acid, Thr or Ser;
R₁ and R₂, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, C₁₂₋₂₀ naphthylalkynyl, COE, or CODE in which E is C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl, provided that when one of R₁ or R₂ is COE or CODE, the other must be H;
R₃ and R₄, independently,ₐᵣₑ H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; and
Y is OR₅ or NR₆R₇ in which each of R₅, R₆ and R₇, independently, is H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; provided that if AA⁸ is Thr or Ser, AA⁶ cannot be Val; for the manufacture of a medicament for use in the treatment of a disease which involves Neuromedin B receptor and does not involve somatostatin receptor.

2. Use of a peptide according to Claim 1, wherein R₃ and R₄ are each H.

3. Use of a peptide according to claim 1, wherein AA⁶ is Nal, Trp, Me-Trp, Bpa, F₅Phe, Phe or 4-X-Phe in which X is halogen, NO₂, CH₃ or OH.

4. Use of a peptide according to claim 1 in which the peptide is selected from 
H2-D-Nal-Cys-Tyr-D-Trp-Lys-Val-Cys-Nal-NH2;
H2-D-Nal-Cys-Tyr-D-Trp-Lys-Nal-Cys-Thr-NH2;
H2-D-Nal-Cys-Tyr-D-Trp-Lys-Nal-Cys-Nal-NH2;
H2-D-Phe-Cys-Tyr-D-Trp-Lys-Val-Cys-Nal-NH2;
H2-D-Trp-Cys-Tyr-D-Trp-Lys-Val-Cys-Nal-NH2;
H2-D-Nal-Cys-Tyr-D-Trp-Lys-Phe-Cys-Nal-NH2; and
H2-D-Phe-Cys-Tyr-D-Trp-Lys-Nal-Cys-Thr-NH2.
 for the manufacture of a medicament for use in the treatment of a disease which involves Neuromedin B receptor and does not involve somatostatin receptor.

5. Use of an octapeptide of the formula: 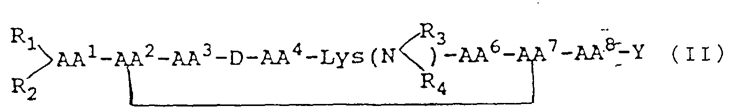 wherein
AA¹ is the D-isomer of an aromatic α-amino acid;
AA² is the L-isomer of Cys;
AA³ is Tyr;
AA⁴ is Trp;
AA⁶ is Thr or Ser;
AA⁷ is the L-isomer of Cys;
AA⁸ is the L-isomer of an aromatic α-amino acid;
R₁ and R₂, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, C₁₂₋₂₀ naphthylalkynyl, COE, or CODE in which E is C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl, provided that when one of R₁ or R₂ is COE or CODE, the other must be H;
R₃ and R₄, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; and
Y is OR₅ or NR₆R₇ in which each of R₅, R₆ and R₇, independently, is H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ napthylalkyl, C₁₂₋₂₀ napthylalkynyl or C₁₂₋₂₀ napthylalkenyl; for the manufacture of a medicament for use in the treatment of a disease which involves Neuromedin B receptor and does not involve somatostatin receptor.

6. Use of a peptide according to Claim 5, wherein AA¹ is D-Phe.

7. Use of a peptide according to Claim 5 in which the peptide is selected from 
H2-D-Phe-Cys-Tyr-D-Trp-Lys-Thr-Cys-Nal-NH2;
H2-D-Phe-Cys-Tyr-D-Trp-Lys(iPr)-Thr-Cys-Nal-NH2;
H2-D-Phe-Cys-Tyr-D-Trp-Lys(diEt)-Thr-Cys-Nal-NH2
H2-D-Phe-Cys-Tyr-D-Trp-Lys-Ser-Cys-Thr-NH2; and
H2-D-Nal-Cys-Tyr-D-Trp-Lys-Thr-Cys-Nal-NH2.
 for the manufacture of a medicament for use in the treatment of a disease which involves Neuromedin B receptor and does not involve somatostatin receptor.

8. Use of an octapeptide of the formula: 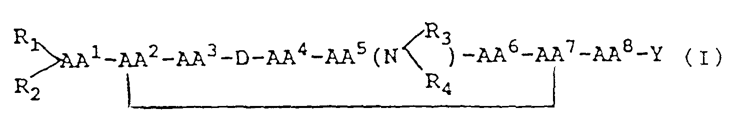 wherein
AA¹ is the D- or L-isomer of an aromatic α-amino acid;
AA² is the D- or L-isomer of Cys;
AA³ is F₅Phe, Phe, or X-Phe in which X is halogen, NO₂, CH₃, or OH;
AA⁴ is Trp, or an aromatic α- amino acid;
AA⁵ is Lys or Orn;
AA⁶ is Leu, Ile, Nle, Val, Nal, Trp, Me-Trp, Bpa, F₅Phe, Phe, or X-Phe in which X is halogen, HO₂, CH₃, or OH;
AA⁷ is the D- or L-isomer of Cys;
AA⁸ is the D- or L-isomer
of an aromatic α-amino acid, Thr or Ser;
R₁ and R₂, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, C₁₂₋₂₀ naphthylalkynyl, COE, or CODE in which E is C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl, provided that when one of R₁ or R₂ is COE or CODE, the other must be H;
R₃ and R₄, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; and
Y is OR₅ or NR₆R₇ in which each of R₅, R₆ and R₇, independently, is H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; provided that AA¹ and AA² cannot both be D-isomers; and further provided that if AA⁸ is Thr or Ser, AA⁶ cannot be Val; and excluding D-Nal-Cys-Tyr-D-Trp-Lys-Val-Cys-Nal-NH2(SS-octa) for the manufacture of a medicament for use in the treatment of a disease which involves Neuromedin B receptor and does not involve somatostatin receptor.

9. Use of a peptide according to Claim 8, wherein AA³ is Tyr.

10. Use of a peptide according to Claim 8 or Claim 9, wherein AA⁴ is Nal.

11. Use of a peptide according to any one of Claims 8, 9 or 10, wherein R₃ and R₄ are each H.

12. Use of a peptide according to Claim 8, wherein AA⁶ is Nal, Trp, Me-Trp, Bpa, F₅Phe, Phe or X-Phe in which X is halogen, NO₂, CH₃ or OH.

13. Use of a peptide according to Claim 8, where AA¹ is a D-isomer and AA⁸ is an L-isomer, or AA¹ is an L-isomer and AA⁸ is a D-isomer.

14. Use of a peptide according to Claim 8 in which the peptide is selected from

H2-D-Nal-Cys-Tyr-D-Trp-Lys-Val-Cys-D-Nal-NH2;
H2-Nal-Cys-Tyr-D-Trp-Lys-Val-Cys-D-Nal-NH2;
H2-D-Nal-D-Cys-Tyr-D-Trp-Lys-Val-Cys-Nal-NH2;
H2-D-Nal-Cys-Tyr-D-Trp-Lys-Val-D-Cys-Nal-NH2;
H2-D-Nal-Cys-Tyr-D-Nal-Lys-Val-Cys-Nal-NH2; and
H2-D-Nal-Cys-Tyr-D-Trp-Orn-Val-Cys-Nal-NH2

15. Use of an Octapeptide of the formula: 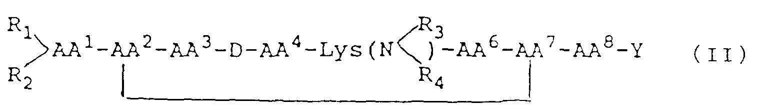 wherein
AA¹ is the D- or L-isomer of an aromatic α-amino acid;
AA² is the D- or L-isomer of Cys;
AA³ is F₅Phe, Phe, or X-Phe in which X is halogen, NO₂, CH₃, or OH;
AA⁴ is Trp, or an aromatic α- amino acid;
AA⁶ is Thr or Ser;
AA⁷ is the D- or L-isomer of Cys;
AA⁸ is the D- or L-isomer
of an aromatic α-amino acid;
R₁ and R₂, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, C₁₂₋₂₀ naphthylalkynyl, COE, or CODE in which E is C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl, provided that when one of R₁ or R₂ is COE or CODE, the other must be H;
R₃ and R₄, independently, are H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ naphthylalkyl, C₁₂₋₂₀ naphthylalkenyl, or C₁₂₋₂₀ naphthylalkynyl; and of
Y is OR₅ or NR₆R₇ in which each of R₅, R₆ and R₇, independently, is H, C₁₋₁₂ alkyl, C₂₋₁₂ alkenyl, C₂₋₁₂ alkynyl, phenyl, naphthyl, C₇₋₁₂ phenylalkyl, C₈₋₁₂ phenylalkenyl, C₈₋₁₂ phenylalkynyl, C₁₁₋₂₀ napthylalkyl, C₁₂₋₂₀ napthylalkenyl or C₁₂₋₂₀ napthylalkynyl; provided that AA¹ and AA² cannot both be D-isomers; for the manufacture of a medicament for use in the treatment of a disease which involves Neuromedin B receptor and does not involve somatostatin receptor.

16. Use of a peptide according to Claim 15, wherein AA¹ is D-Phe.

17. Use of a peptide according to Claim 15 or Claim 16, wherein AA³ is Tyr.

18. Use of a peptide according to any one of Claims 15 16 or 17, wherein AA⁴ is Trp.

19. Use of a peptide according to Claim 15, wherein AA¹ is a D-isomer and AA⁸ is a L-isomer, or AA¹ is a L-isomer and AA⁸ is a D-isomer.

20. Use of a peptide according to Claim 15 in which the peptide is selected from

H2-D-Nal-D-Cys-Tyr-D-Trp-Lys-Thr-Cys-Nal-NH2; and
H2-D-Nal-Cys-Phe-D-Trp-Lys-Thr-Cys-Nal-NH2.

## Patentansprüche

1. Verwendung eines Octapeptids der Formel: 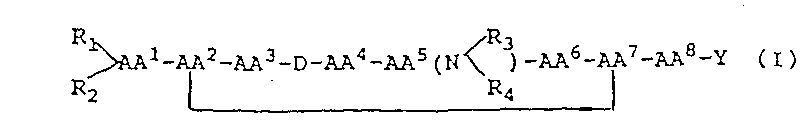 wobei
AA¹ das D-Isomer einer aromatischen α-Aminosäure ist;
AA² das L-Isomer von Cys ist;
AA³ Tyr ist;
AA⁴ Trp ist;
AA⁵ Lys ist;
AA⁶ Leu, Ile, Nle, Val, Nal, Trp, Me-Trp, Bpa, F₅Phe, Phe oder 4-X-Phe ist, wobei X Halogen, NO₂, CH₃ oder OH ist;
AA⁷ das L-Isomer von Cys ist;
AA⁸ das L-Isomer einer aromatischen α-Aminosäure, Thr oder Ser ist;
R₁ und R₂ unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl, C₁₂₋₂₀ Naphthylalkynyl, COE oder COOE sind, wobei E C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl ist, vorausgesetzt, dass, wenn R₁ oder R₂ COE oder COOE ist, das andere H sein muss;
R₃ und R₄ unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl sind; und
Y OR₅ oder NR₆R₇ ist , wobei R₅, R₆ und R₇ jeweils unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl sind, vorausgesetzt, dass,
wenn AA⁸ Thr oder Ser ist, AA⁶ nicht Val sein kann; zur Herstellung eines Medikamentes zur Verwendung bei der Behandlung einer Krankheit, die mit dem Neuromedin-B-Rezeptor, aber nicht mit dem Somatostatin-Rezeptor verbunden ist.

2. Verwendung eines Peptids nach Anspruch 1, wobei R₃ und R₄ jeweils H sind.

3. Verwendung eines Peptids nach Anspruch 1, wobei AA⁶ Nal, Trp, Me-Trp, Bpa, F₅Phe, Phe oder 4-X-Phe ist, wobei X Halogen, NO₂, CH₃ oder OH ist.

4. Verwendung eines Peptids nach Anspruch 1, wobei das Peptid ausgewählt ist aus 
H2-D-Nal-Cys-Tyr-D-Trp-Lys-Val-Cys-Nal-NH2;
H2-D-Nal-Cys-Tyr-D-Trp-Lys-Nal-Cys-Thr-NH2;
H2-D-Nal-Cys-Tyr-D-Trp-Lys-Nal-Cys-Nal-NH2;
H2-D-Phe-Cys-Tyr-D-Trp-Lys-Val-Cys-Nal-NH2;
H2-D-Trp-Cys-Tyr-D-Trp-Lys-Val-Cys-Nal-NH2;
H2-D-Nal-Cys-Tyr-D-Trp-Lys-Phe-Cys-Nal-NH2; und
H2-D-Phe-Cys-Tyr-D-Trp-Lys-Nal-Cys-Thr-NH2;
 zur Herstellung eines Medikamentes zur Verwendung bei der Behandlung einer Krankheit, die mit dem Neuromedin-B-Rezeptor, aber nicht mit dem Somatostatin-Rezeptor verbunden ist.

5. Verwendung eines Octapeptids der Formel: 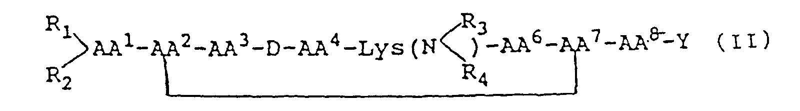 wobei
AA¹ das D-Isomer einer aromatischen α-Aminosäure ist;
AA² das L-Isomer von Cys ist;
AA³ Tyr ist;
AA⁴ Trp ist;
AA⁶ Thr oder Ser ist;
AA⁷ das L-Isomer von Cys ist;
AA⁸ das L-Isomer einer aromatischen α-Aminosäure ist;
R₁ und R₂ unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl, C₁₂₋₂₀ Naphthylalkynyl, COE oder COOE sind, wobei E C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl ist, vorausgesetzt, dass, wenn R₁ oder R₂ COE oder CODE ist, das andere H sein muss;
R₃ und R₄ unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl sind; und
Y OR₅ oder NR₆R₇ ist, wobei R₅, R₆ und R₇ jeweils unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkynyl oder C₁₂₋₂₀ Naphthylalkenyl sind; zur Herstellung eines Medikamentes zur Verwendung bei der Behandlung einer Krankheit, die mit dem Neuromedin-B-Rezeptor, aber nicht mit dem Somatostatin-Rezeptor verbunden ist.

6. Verwendung eines Peptids nach Anspruch 5, wobei AA¹ D-Phe ist.

7. Verwendung eines Peptids nach Anspruch 5, wobei das Peptid ausgewählt ist aus 
H2-D-Phe-Cys-Tyr-D-Trp-Lys-Thr-Cys-Nal-NH2;
H2-D-Phe-Cys-Tyr-D-Trp-Lys(iPr)-Thr-Cys-Nal-NH2;
H2-D-Phe-Cys-Tyr-D-Trp-Lys(diEt)-Thr-Cys-Nal-NH2;
H2-D-Phe-Cys-Tyr-D-Trp-Lys-Ser-Cys-Thr-NH2; und
H2-D-Nal-Cys-Tyr-D-Trp-Lys-Thr-Cys-Nal-NH2,
 zur Herstellung eines Medikamentes zur Verwendung bei der Behandlung einer Krankheit, die mit dem Neuromedin-B-Rezeptor, aber nicht mit dem Somatostatin-Rezeptor verbunden ist.

8. Verwendung eines Octapeptids der Formel: 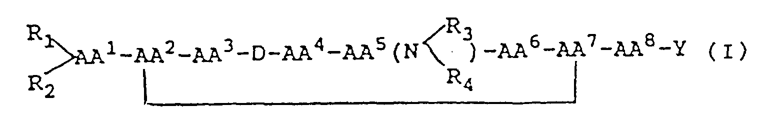 wobei
AA¹ das D- oder L-Isomer einer aromatischen α-Aminosäure ist;
AA² das D- oder L-Isomer von Cys ist;
AA³ F₅Phe, Phe oder X-Phe ist, wobei X Halogen, NO₂, CH₃ oder OH ist;
AA⁴ Trp oder eine aromatische α-Aminosäure ist;
AA⁵ Lys oder Orn ist;
AA⁶ Leu, Ile, Nle, Val, Nal, Trp, Me-Trp, Bpa, F₅Phe, Phe oder X-Phe ist, wobei X Halogen, NO₂, CH₃ oder OH ist;
AA⁷ das D- oder L-Isomer von Cys ist;
AA⁸ das D- oder L-Isomer einer aromatischen α-Aminosäure, Thr oder Ser ist;
R₁ und R₂ unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl, C₁₂₋₂₀ Naphthylalkynyl, COE oder COOE sind, wobei E C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl ist, vorausgesetzt, dass, wenn R₁ oder R₂ COE oder COOE ist, das andere H sein muss;
R₃ und R₄ unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl sind; und
Y OR₅ oder NR₆R₇ ist, wobei R₅, R₆ und R₇ jeweils unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl sind, vorausgesetzt, dass AA¹ und AA² nicht beide D-Isomere sein können; und dass, wenn AA⁸ Thr oder Ser ist, AA⁶ nicht Val sein kann; und unter Ausschluss von D-Nal-Cys-Tyr-D-Trp-Lys-Val-Cys-Nal-NH₂(SS-octa) zur Herstellung eines Medikamentes zur Verwendung bei der Behandlung einer Krankheit, die mit dem Neuromedin-B-Rezeptor, aber nicht mit dem Somatostatin-Rezeptor verbunden ist.

9. Verwendung eines Peptids nach Anspruch 8, wobei AA³ Tyr ist.

10. Verwendung eines Peptids nach Anspruch 8 oder Anspruch 9, wobei AA⁴ Nal ist.

11. Verwendung eines Peptids nach einem der Ansprüche 8, 9 oder 10, wobei R₃ und R₄ jeweils H sind.

12. Verwendung eines Peptids nach Anspruch 8, wobei AA⁶ Nal, Trp, Me-Trp, Bpa, F₅Phe, Phe oder X-Phe ist, wobei X Halogen, NO₂, CH₃ oder OH ist.

13. Verwendung eines Peptids nach Anspruch 8, wobei AA¹ ein D-Isomer und AA⁸ ein L-Isomer oder AA¹ ein L-Isomer und AA⁸ ein D-Isomer ist.

14. Verwendung eines Peptids nach Anspruch 8, wobei das Peptid ausgewählt ist aus

H2-D-Nal-Cys-Tyr-D-Trp-Lys-Val-Cys-D-Nal-NH2;
H2-Nal-Cys-Tyr-D-Trp-Lys-Val-Cys-D-Nal-NH2;
H2-D-Nal-D-Cys-Tyr-D-Trp-Lys-Val-Cys-Nal-NH2;
H2-D-Nal-Cys-Tyr-D-Trp-Lys-Val-D-Cys-Nal-NH2;
H2-D-Nal-Cys-Tyr-D-Nal-Lys-Val-Cys-Nal-NH2; und
H2-D-Nal-Cys-Tyr-D-Trp-Orn-Val-Cys-Nal-NH2.

15. Verwendung eines Octapeptids der Formel: 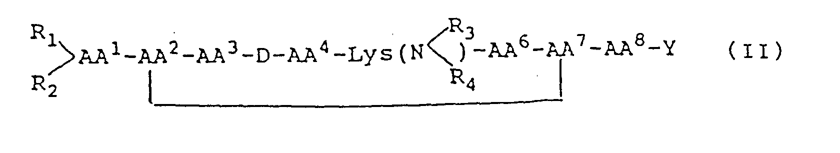 wobei
AA¹ das D- oder L-Isomer einer aromatischen α-Aminosäure ist;
AA² das D- oder L-Isomer von Cys ist;
AA³ F₅Phe, Phe oder X-Phe ist, wobei X Halogen, NO₂, CH₃ oder OH ist;
AA⁴ Trp oder eine aromatische α-Aminosäure ist;
AA⁶ Thr oder Ser ist;
AA⁷ das D- oder L-Isomer von Cys ist;
AA⁸ das D- oder L-Isomer einer aromatischen α-Aminosäure ist;
R₁ und R₂ unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl, C₁₂₋₂₀ Naphthylalkynyl, COE oder COOE sind, wobei E C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl ist, vorausgesetzt, dass, wenn R₁ oder R₂ COE oder COOE ist, das andere H sein muss;
R₃ und R₄ unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl sind; und
Y OR₅ oder NR₆R₇ ist, wobei R₅, R₆ und R₇ jeweils unabhängig H, C₁₋₁₂ Alkyl, C₂₋₁₂ Alkenyl, C₂₋₁₂ Alkynyl, Phenyl, Naphthyl, C₇₋₁₂ Phenylalkyl, C₈₋₁₂ Phenylalkenyl, C₈₋₁₂ Phenylalkynyl, C₁₁₋₂₀ Naphthylalkyl, C₁₂₋₂₀ Naphthylalkenyl oder C₁₂₋₂₀ Naphthylalkynyl sind, vorausgesetzt, dass, AA¹ und AA² nicht beide D-Isomere sein können; zur Herstellung eines Medikamentes zur Verwendung bei der Behandlung einer Krankheit, die mit dem Neuromedin-B-Rezeptor, aber nicht mit dem Somatostatin-Rezeptor verbunden ist.

16. Verwendung eines Peptids nach Anspruch 15, wobei AA¹ D-Phe ist.

17. Verwendung eines Peptids nach Anspruch 15 oder Anspruch 16, wobei AA³ Tyr ist.

18. Verwendung eines Peptids nach einem der Ansprüche 15, 16 oder 17, wobei AA⁴ Trp ist.

19. Verwendung eines Peptids nach Anspruch 15, wobei AA¹ ein D-Isomer und AA⁸ ein L-Isomer oder AA¹ ein L-Isomer und AA⁸ ein D-Isomer ist.

20. Verwendung eines Peptids nach Anspruch 15, wobei das Peptid ausgewählt ist aus

H₂-D-Nal-D-Cys-Tyr-D-Trp-Lys-Thr-Cys-Nal-NH₂; und
H₂-D-Nal-Cys-Phe-D-Trp-Lys-Thr-Cys-Nal-NH₂.

## Revendications

1. Utilisation d'un octapeptide de formule : 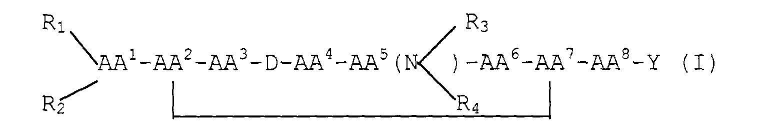 dans laquelle
AA¹ représente l'isomère D d'un acide aminé α aromatique ;
AA² représente l'isomère L de Cys ;
AA³ représente Tyr ;
AA⁴ représente Trp ;
AA⁵ représente Lys ;
AA⁶ représente Leu, Ile, Nle, Val, Nal, Trp, Me-Trp, Bpa, F₅Phe, Phe ou 4-X-Phe dans lequel X représente un halogène, NO₂, CH₃ ou OH ;
AA⁷ représente l'isomère L de Cys ;
AA⁸ représente l'isomère L d'un acide aminé α aromatique, Thr ou Ser ;
R₁ et R₂, indépendamment, représentent H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀, un naphtylalkynyle en C₁₂₋₂₀, COE ou COOE dans lequel E représente un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀, à condition que lorsque l'un parmi R₁ et R₂ représente COE ou CODE, l'autre doit représenter H ;
R₃ et R₄, indépendamment, représentent H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀; et
Y représente OR₅ ou NR₆R₇ dans lequel R₅, R₆ et R₇ représentent chacun, indépendamment, H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀ ; à condition que
si AA⁸ représente Thr ou Ser, AA⁶ ne peut pas représenter Val ; pour la fabrication d'un médicament pour une utilisation dans le traitement d'une maladie qui implique le récepteur de la neuromédine B et qui n'implique pas le récepteur de la somatostatine.

2. Utilisation d'un peptide selon la revendication 1, dans lequel R₃ et R₄ représentent chacun H.

3. Utilisation d'un peptide selon la revendication 1, dans lequel AA⁶ représente Nal, Trp, Me-Trp, Bpa, F₅Phe, Phe ou 4-X-Phe dans lequel X représente un halogène, NO₂, CH₃ ou OH.

4. Utilisation d'un peptide selon la revendication 1, dans laquelle le peptide est choisi parmi 
H2-D-Nal-Cys-Tyr-D-Trp-Lys-Val-Cys-Nal-NH2 ;
H2-D-Nal-Cys-Tyr-D-Trp-Lys-Nal-Cys-Thr-NH2 ;
H2-D-Nal-Cys-Tyr-D-Trp-Lys-Nal-Cys-Nal-NH2 ;
H2-D-Phe-Cys-Tyr-D-Trp-Lys-Val-Cys-Nal-NH2 ;
H2-D-Trp-Cys-Tyr-D-Trp-Lys-Val-Cys-Nal-NH2 ;
H2-D-Nal-Cys-Tyr-D-Trp-Lys-Phe-Cys-Nal-NH2 ; et
H2-D-Phe-Cys-Tyr-D-Trp-Lys-Nal-Cys-Thr-NH2 ;
 pour la fabrication d'un médicament pour une utilisation dans le traitement d'une maladie qui implique le récepteur de la neuromédine B et qui n'implique pas le recepteur de la somatostatine.

5. Utilisation d'un octapeptide de formule : 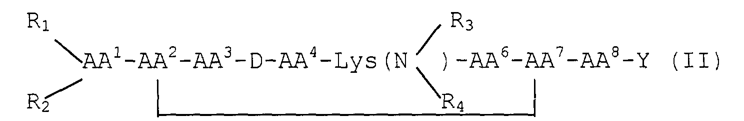 dans laquelle
AA¹ représente l'isomère D d'un acide aminé α aromatique ;
AA² représente l'isomère L de Cys ;
AA³ représente Tyr ;
AA⁴ représente Trp ;
AA⁶ représente Thr ou Ser ;
AA⁷ représente l'isomère L de Cys ;
AA⁸ représente l'isomère L d'un acide aminé α aromatique ;
R₁ et R₂, indépendamment, représentent H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀, un naphtylalkynyle en C₁₂₋₂₀, COE ou COOE dans lequel E représente un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀, à condition que lorsque l'un parmi R₁ et R₂ représente COE ou CODE, l'autre doit représenter H ;
R₃ et R₄, indépendamment, représentent H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀ ; et
Y représente OR₅ ou NR₆R₇ dans lequel R₅, R₆ et R₇ représentent chacun, indépendamment, H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkynyle en C₁₂₋₂₀ ou un naphtylalkényle en C₁₂₋₂₀ ; pour la fabrication d'un médicament pour une utilisation dans le traitement d'une maladie qui implique le récepteur de la neuromédine B et qui n'implique pas le récepteur de la somatostatine.

6. Utilisation d'un peptide selon la revendication 5, dans lequel AA¹ représente D-Phe.

7. Utilisation d'un peptide selon la revendication 5, dans laquelle le peptide est choisi parmi 
H2-D-Phe-Cys-Tyr-D-Trp-Lys-Thr-Cys-Nal-NH2;
H2-D-Phe-Cys-Tyr-D-Trp-Lys(iPr)-Thr-Cys-Nal-NH2;
H2-D-Phe-Cys-Tyr-D-Trp-Lys(diEt)-Thr-Cys-Nal-NH2
H2-D-Phe-Cys-Tyr-D-Trp-Lys-Ser-Cys-Thr-NH2; and
H2-D-Nal-Cys-Tyr-D-Trp-Lys-Thr-Cys-Nal-NH2.
 pour la fabrication d'un médicament pour une utilisation dans le traitement d'une maladie qui implique le récepteur de la neuromédine B et qui n'implique pas le récepteur de la somatostatine.

8. Utilisation d'un octapeptide de formule : 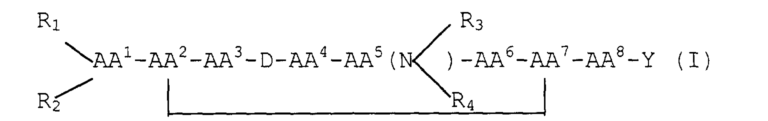 dans laquelle
AA¹ représente l'isomère D ou L d'un acide aminé α aromatique ;
AA² représente l'isomère D ou L de Cys ;
AA³ représente F₅Phe, Phe ou X-Phe dans lequel X représente un halogène, NO₂, CH₃ ou OH ;
AA⁴ représente Trp ou un acide aminé α aromatique ;
AA⁵ représente Lys ou Orn;
AA⁶ représente Leu, Ile, Nle, Val, Nal, Trp, Me-Trp, Bpa, F₅Phe, Phe ou X-Phe dans lequel X représente un halogène, NO₂, CH₃ ou OH ;
AA⁷ représente l'isomère D ou L de Cys ;
AA⁸ représente l'isomère D ou L d'un acide aminé α aromatique, Thr ou Ser ;
R₁ et R₂, indépendamment, représentent H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀, un naphtylalkynyle en C₁₂₋₂₀, COE ou COOE dans lequel E représente un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀, à condition que lorsque l'un parmi R₁ et R₂ représente COE ou COOE, l'autre doit représenter H ;
R₃ et R₄, indépendamment, représentent H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀ ; et
Y représente OR₅ ou NR₆R₇ dans lequel R₅, R₆ et R₇ représentent chacun, indépendamment, H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀ ; à condition que AA¹ et AA² ne peuvent représenter tous deux des isomères D ; et en outre à condition que si AA⁸ représente Thr ou Ser, AA⁶ ne peut pas représenter Val ; et à l'exclusion de D-Nal-Cys-Tyr-D-Trp-Lys-Val-Cys-Nal-NH2(SS-octa) pour la fabrication d'un médicament pour une utilisation dans le traitement d'une maladie qui implique le récepteur de la neuromédine B et qui n'implique pas le récepteur de la somatostatine.

9. Utilisation d'un peptide selon la revendication 8, dans lequel AA³ représente Tyr.

10. Utilisation d'un peptide selon la revendication 8 ou la revendication 9, dans lequel AA⁴ représente Nal.

11. Utilisation d'un peptide selon l'une quelconque des revendications 8, 9 ou 10, dans lequel R₃ et R₄ représentent chacun H.

12. Utilisation d'un peptide selon la revendication 8, dans lequel AA⁶ représente Nal, Trp, Me-Trp, Bpa, F₅Phe, Phe ou X-Phe dans lequel X représente un halogène, NO₂, CH₃ ou OH.

13. Utilisation d'un peptide selon la revendication 8, dans lequel AA¹ représente un isomère D et AA⁸. représente un isomère L, ou AA¹ représente un isomère L et AA⁸ représente un isomère D.

14. Utilisation d'un peptide selon la revendication 8. dans laquelle le peptide est choisi parmi

H2-D-Nal-Cys-Tyr-D-Trp-Lys-Val-Cys-D-Nal-NH2 ;
H2-Nal-Cys-Tyr-D-Trp-Lys-Val-Cys-D-Nal-NH2 ;
H2-D-Nal-D-Cys-Tyr-D-Trp-Lys-Val-Cys-Nal-NH2 ;
H2-D-Nal-Cys-Tyr-D-Trp-Lys-Val-D-Cys-Nal-NH2 ;
H2-D-Nal-Cys-Tyr-D-Nal-Lys-Val-Cys-Nal-NH2 ; et
H2-D-Nal-Cys-Tyr-D-Trp-Orn-Val-Cys-Nal-NH2 ;

15. Utilisation d'un octapeptide de formule : 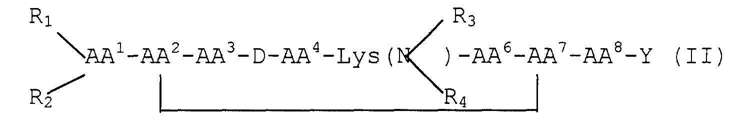 dans laquelle
AA¹ représente l'isomère D ou L d'un acide aminé α aromatique ;
AA² représente l'isomère D ou L de Cys ;
AA³ représente F₅Phe, Phe ou X-Phe dans lequel X représente un halogène, NO₂, CH₃ ou OH ;
AA⁴ représente Trp ou un acide aminé α aromatique;
AA⁶ représente Thr ou Ser ;
AA⁷ représente l'isomère D ou L de Cys ;
AA⁸ représente l'isomère D ou L d'un acide aminé α aromatique ;
R₁ et R₂, indépendamment, représentent H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀, un naphtylalkynyle en C₁₂₋₂₀, COE ou CODE dans lequel E représente un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀, à condition que lorsque l'un parmi R₁ et R₂ représente COE ou COOE, l'autre doit représenter H ;
R₃ et R₄, indépendamment, représentent H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀; et
Y représente OR₅ ou NR₆R₇ dans lequel R₅, R₆ et R₇ représentent chacun, indépendamment, H, un alkyle en C₁₋₁₂, un alkényle en C₂₋₁₂, un alkynyle en C₂₋₁₂, un phényle, un naphtyle, un phénylalkyle en C₇₋₁₂, un phénylalkényle en C₈₋₁₂, un phénylalkynyle en C₈₋₁₂, un naphtylalkyle en C₁₁₋₂₀, un naphtylalkényle en C₁₂₋₂₀ ou un naphtylalkynyle en C₁₂₋₂₀ ; à condition que AA¹ et AA² ne peuvent représenter tous deux des isomères D ; pour la fabrication d'un médicament pour une utilisation dans le traitement d'une maladie qui implique le récepteur de la neuromédine B et qui n'implique pas le récepteur de la somatostatine.

16. Utilisation d'un peptide selon la revendication 15, dans lequel AA¹ représente D-Phe.

17. Utilisation d'un peptide selon la revendication 15 ou la revendication 16, dans lequel AA³ représente Tyr.

18. Utilisation d'un peptide selon l'une quelconque des revendications 15, 16 ou 17, dans lequel AA⁴ représente Trp.

19. Utilisation d'un peptide selon la revendication 15, dans lequel AA¹ représente un isomère D et AA⁸ représente un isomère L, ou AA¹ représente un isomère L et AA⁸ représente un isomère D.

20. Utilisation d'un peptide selon la revendication 15, dans laquelle le peptide est choisi parmi
